# EUROPEAN PATENT APPLICATION

(11) **EP 3 474 219 A1**
(43) Date of publication of application: **24.04.2019**
(21) Application number: 17820105.9
(22) Date of filing: 26.06.2017
(51) Int. Cl.: G06Q 50/22, A61G 12/00

(54) **NURSING/CARE ITEM INPUT DEVICE, NURSING/CARE ITEM INPUT METHOD, AND NURSING/CARE ITEM MANAGEMENT SYSTEM**

(30) Priority: 28.06.2016 JP 2016127261
(71) Applicant: Konica Minolta, Inc., Tokyo 100-7015 (JP)
(72) Inventor: NISHIKADO, Masashi, Tokyo 100-7015 (JP); UESAKA, Takeshi, Tokyo 100-7015 (JP)
(74) Representative: Hoffmann Eitle
(86) International application number: PCT/JP2017/023429
(87) International publication number: WO 2018/003750

(57) **Abstract**

When inputting results of nursing/care regarding a plurality of nursing/care items that are determined in advance for each type of nursing/care, a display screen including the plurality of nursing/care items is displayed on a display unit, an operation input by a user is accepted, an input screen is displayed on the display unit, when one nursing/care item is selected as an input target from among the plurality of cursing/care items by the user in a state where the display screen is displayed on the display unit, the input screen for inputting a result of nursing/care regarding the selected one nursing/care item, one of the plurality of nursing/care items is nursing/care provision time indicative of the time when nursing/care is provided, a screen is displayed as the input screen on the display unit, when nursing/care provision time is selected from among the plurality of nursing/care items as an input target, the screen indicating a plurality of different elapsed times dating back from current time as options of the nursing/care provision time.

## Description

### Technical Field

The present invention relates to a nursing/care item input device which is used to input results of nursing/care regarding a plurality of nursing/care items determined in advance for each type of nursing/care, the nursing/care to be provided for a monitored person as a monitoring target, the nursing/care including at least one of nursing and care, a nursing/care item input method, and a nursing/care item management system equipped with the nursing/care item input device.

### Background Art

Our country (Japan) has become an aging society, in more detail, a super aging society where an elderly population rate as a rate of population aged 65 or over to a total population exceeds 21 % due to improved standards of living, improved hygienic environment, and improved standards of medical care, etc. which were caused by postwar rapid economic growth. According to the statistics as of September 2013, the elderly population was about 31.86 million and the elderly population rate was 25.0%, one in four of which was elderly people. Then, some forecast that in 2035, the elderly population will be about 37.41 million, one in three of which will be elderly people (Statics Bureau, Ministry of Internal Affairs and Communications of Japan). In such an aging society, nursing-requiring persons who require nursing or care (nursing-requiring/care-requiring persons) are expected to be increased in number due to sickness, injury, aging, or the like more than nursing-requiring/care-requiring persons in an ordinary, not aging, society. Our country is also a birth rate declining society with the total fertility rate as of, for example, 2013 of 1.43. Therefore, elderly-elderly care has occurred, the care of an aged nursing-requiring/care-requiring person by an aged family member (spouse, child, brother, sister).

Nursing-requiring/care-requiring persons stay in such facilities as hospitals, welfare facilities for the aged (short stay facilities for the elderly, elderly nursing home, special elderly nursing home, etc. under Japanese Law), and the like to receive nursing or care. In such elderly facilities, situations could occur that nursing-requiring/care-requiring persons fall off from beds or tumble during walking to get injury, and those persons get out of beds to wander, and like. Such situations have to be handled as soon as possible. Setting such situations aside might develop into further serious situations. In the facilities, therefore, nurses or care workers (nursing/care staff) periodically patrol to see if nursing-requiring/care-requiring persons are safe or how they are. Then, nurses, care workers, and the like record results of patrolling as a nursing record or a care record (a nursing/care record).

However, the increase in the number of nursing/care staffs cannot catch up with the increase in the number of nursing-requiring/care-requiring persons, so that the nursing industry and the care industry have chronic shortage of labor. Further, as compared with a time zone of day shift, in a time zone of quasi-night shift or night shift, less nurses, care workers, and the like work with an increased work load per worker, and therefore mitigation of the work load is demanded. Additionally, situations of elderly-elderly care are not exceptions in the facilities, and caring of elderly nursing-requiring/care-requiring persons by elderly nursing/care staffs is frequently seen. Since aged person lose physical strength in general, the staffs, though healthy, have nursing/care loads becoming heavier as compared with young nurses and the like and become slow in action and determination.

For mitigating such labor shortage and loads on nursing/care staffs, techniques for complementing nursing work and care work are required. As one of such techniques, for example, Patent Literature 1 discloses a technique of facilitating input for recording nursing/care conducted with respect to a monitored person as a monitoring target by using a mobile information terminal.

### Citation List

### Patent Literature

Patent Literature 1: Unexamined Japanese Patent Publication No. 2014-87524

### Summary of Invention

For recording nursing/care provided for a monitored person, it is generally necessary to input results of nursing/care regarding a plurality of nursing/care items determined in advance for each type of nursing/care to be provided. However, further simplification of input work is demanded for inputting results of nursing/care regarding a plurality of nursing/care items at site while executing nursing/care for a monitored person. In particular, the time when nursing/care was provided should be input as a result of nursing/care regarding a nursing/care item regardless of the type of nursing/care. Accordingly, improvement is strongly demanded in particular for the work of inputting time of provision of nursing/care. However, the above Patent Literature 1 does not satisfactorily consider simplification of work of inputting the time when nursing/care was provided.

An object of the present invention, which has been made in consideration of the above circumstances, is to provide a nursing/care item input device, a nursing/care item input method, and a nursing/care item management system which realize simplification of work of inputting the time when nursing/care was provided.

In order to realize at least one of the above objects, a nursing/care item input device reflecting one aspect of the present invention is
a nursing/care item input device which is used to input results of nursing/care regarding a plurality of nursing/care items determined in advance for each type of nursing/care, the nursing/care to be provided for a monitored person as a monitoring target and including at least one of nursing and care, wherein
the nursing/care item input device:
displays a display screen including the plurality of nursing/care items on a display unit;
accepts operation input by a user;
displays on the display unit, when one nursing/care item is selected as an input target from the plurality of nursing/care items by the user using the operation input unit in a state where the display screen is displayed on the display unit, an input screen for inputting a result of nursing/care regarding the selected one nursing/care item, one of the plurality of nursing/care items being nursing/care provision time indicative of a time when the nursing/care is provided; and
displays on the display unit, when the nursing/care provision time is selected as an input target from the plurality of nursing/care items, a screen indicating a plurality of different elapsed times dating back from current time as options of the nursing/care provision time as the input screen.

### Brief Description of Drawings

Advantages and features obtained by one or more embodiments of the present invention will be more fully understood from the following detailed description and accompanying drawings which are provided only for the purpose of explanation and are therefore not intended to define a boundary of the present invention.

FIG. 1 is a diagram showing a configuration of a monitored person monitoring system in an embodiment.
FIG. 2 is a diagram showing a configuration of a management server device of the monitored person monitoring system.
FIG. 3 is a diagram showing a configuration of a server side monitoring information table stored in the management server device.
FIG. 4 is a diagram showing a configuration of an inter-device information table stored in the management server device.
FIG. 5 is a diagram showing a configuration of a server side sensor information table stored in the management server device.
FIG. 6 is a diagram showing a configuration of a form information table stored in the management server device.
FIG. 7 is a diagram showing a configuration of a mobile terminal device in the monitored person monitoring system.
FIG. 8 is a view showing one example of a standby screen displayed on the mobile terminal device.
FIG. 9 is a view showing one example of a monitoring information screen displayed on the mobile terminal device.
FIG. 10 is a view showing one example of a nurse call reception screen displayed on the mobile terminal device.
FIG. 11 is a flow chart showing operation of selecting a recording form by manual operation in the mobile terminal device.
FIG. 12 is a flow chart showing automatic operation of selecting a recording form in the mobile terminal device.
FIG. 13 is a flow chart showing automatic operation of selecting a recording form in the management server device.
FIG. 14 is a view showing one example of a submenu screen displayed on the mobile terminal device.
FIG. 15 is a view showing one example of a target person selection screen displayed on the mobile terminal device.
FIG. 16 is a view showing one example of a form selection screen displayed on the mobile terminal device.
FIG. 17 is a view showing one example of a meal care recording form screen displayed on the mobile terminal device.
FIG. 18 is a view showing one example of a morning care recording form screen displayed on the mobile terminal device.
FIG. 19 is a view showing one example of an excretion care recording form screen displayed on the mobile terminal device.
FIG. 20 is a view showing one example of a food intake selection screen displayed on the mobile terminal device.
FIG. 21 is a view showing one example of a text input screen displayed on the mobile terminal device.
FIG. 22 is a view showing one example of a time selection screen displayed on the mobile terminal device.
FIG. 23 is a view showing one example of input of care provision time on the meal care recording form screen displayed on the mobile terminal device.
FIG. 24 is a view showing one example of a time setting screen displayed on the mobile terminal device.
FIG. 25 is a flow chart showing operation of inputting the care provision time in the mobile terminal device.
FIG. 26 is a view showing one example of a presence/absence input screen displayed on the mobile terminal device.
FIG. 27 is a view showing one example of a care plan managed by the management server device.

### Description of Embodiments

In the following, one or more embodiments of the present invention will be described with reference to the drawings. However, the scope of the present invention is not limited to the disclosed embodiments. Components given the same reference symbols in the drawings represent the same components, description of which is appropriately omitted. In the present specification, a component is represented by a reference symbol without a subscript when generically referred to, and a reference symbol with a subscript is used to indicate an individual component.

A monitored person monitoring system in the embodiment monitors a monitored person Ob as a monitoring target to be monitored (in other words, monitors an observed person Ob as an observation target to be observed), and includes a terminal device and a monitored person monitoring device communicably connected to the terminal device. The monitored person monitoring device in the monitored person monitoring system detects a predetermined event (e.g. a predetermined event to be coped with) related to a monitored person Ob and notifies the terminal device of the event. More specifically, the monitored person monitoring device includes a communication unit which conducts communication and a detection unit which detects a predetermined event related to the monitored person Ob and notifies the terminal device of the event. And, in the embodiment, for supporting a nurse in recording nursing of the monitored person Ob or a care worker in recording care of the monitored person Ob, the monitored person monitoring device further includes a form information storage portion for storing a plurality of recording forms of a predetermined format prepared in advance according to the type of the nursing/care for inputting nursing/care contents including at least one of nursing and care as a nursing/care record, and a form distribution processing portion which selects, when the communication unit receives a communication signal (form request communication signal) containing a status parameter indicative of a predetermined status in a transmission source and an instruction for requesting a recording form, a recording form from among the plurality of recording forms stored in the form information storage portion according to the status parameter contained in the received form request communication signal and transmits a communication signal (form return communication signal) containing the selected recording form to the transmission source by the communication unit. Preferably, the form information storage portion stores the plurality of recording forms such that any one of the plurality of recording forms is correlated with the status parameter. Although such a monitored person monitoring device can be integrally formed by one device, the monitored person monitoring device in the embodiment, which includes a sensor device and a management server device (central processing unit) communicably connected to the sensor device and the terminal device, is configured of two kinds of separate devices. The sensor device detects the predetermined event related to the monitored person Ob and notifies (informs, transmits) the management server device of the event. Upon reception of the notification from the sensor device, the management server device manages the predetermined event and re-notifies (re-informs, re-transmits) a predetermined terminal device correlated with the sensor device of the predetermined event. While the terminal device can be one kind of device, the terminal device in the present embodiment includes two kinds of devices, a fixed terminal device and a mobile terminal device. The fixed terminal device and the mobile terminal device mainly differ in that while the fixed terminal device is fixedly operated, the mobile terminal device is operated being carried by a monitor (user) such as, for example, a nurse or a care worker, and since the fixed terminal device and the mobile terminal device are substantially the same, the following description of the embodiment will be mainly made of the mobile terminal device.

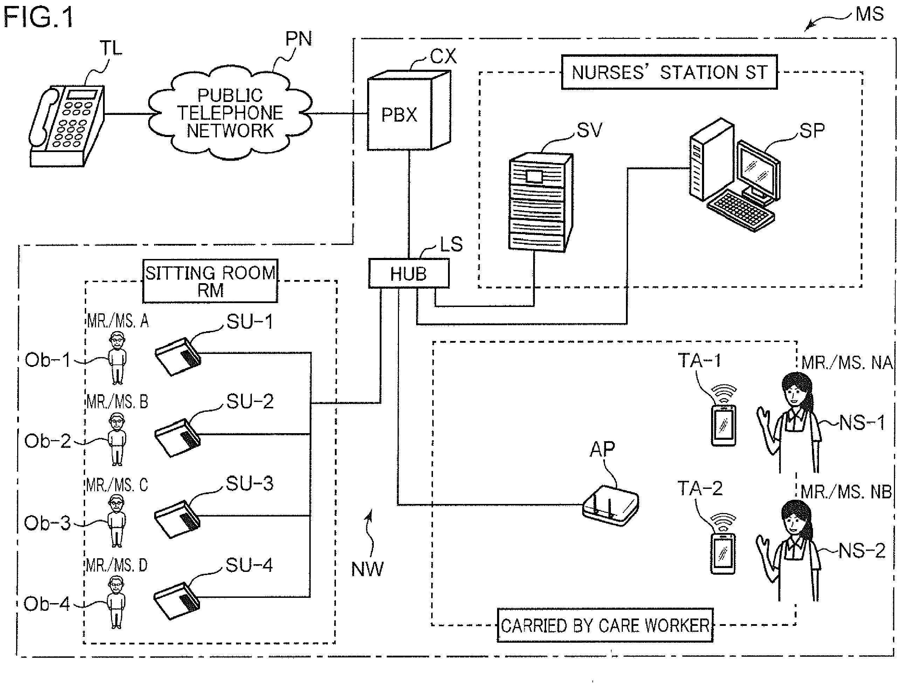

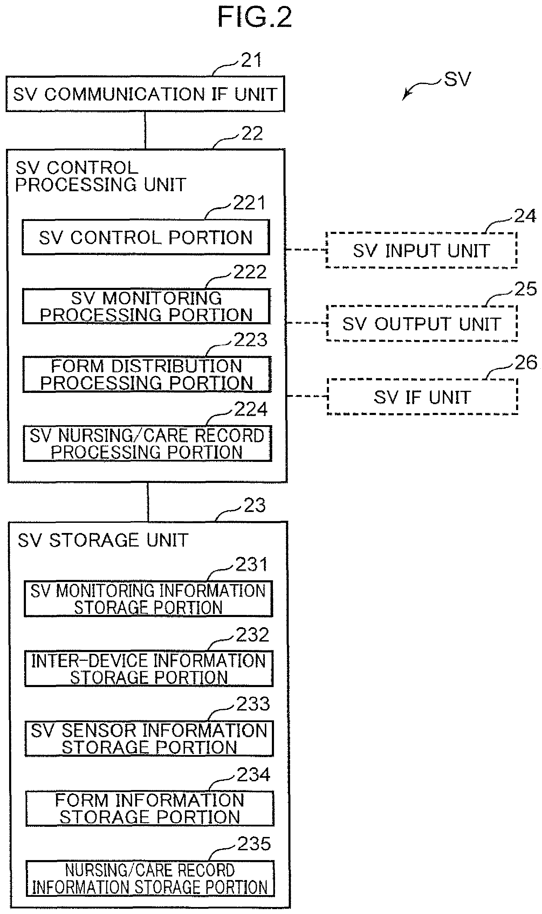

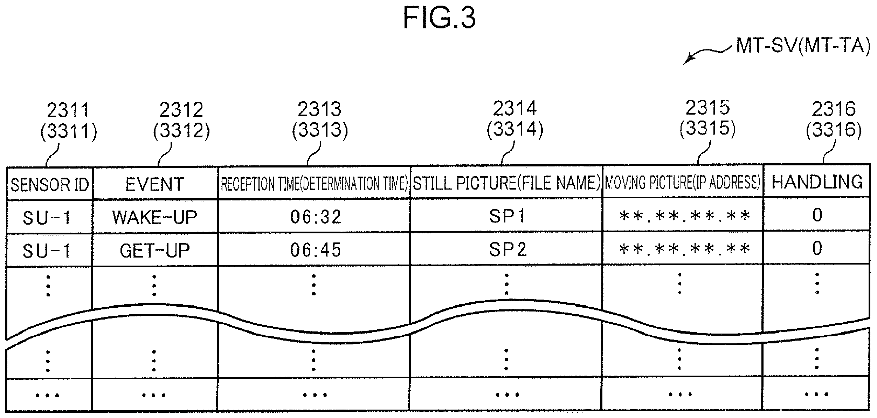

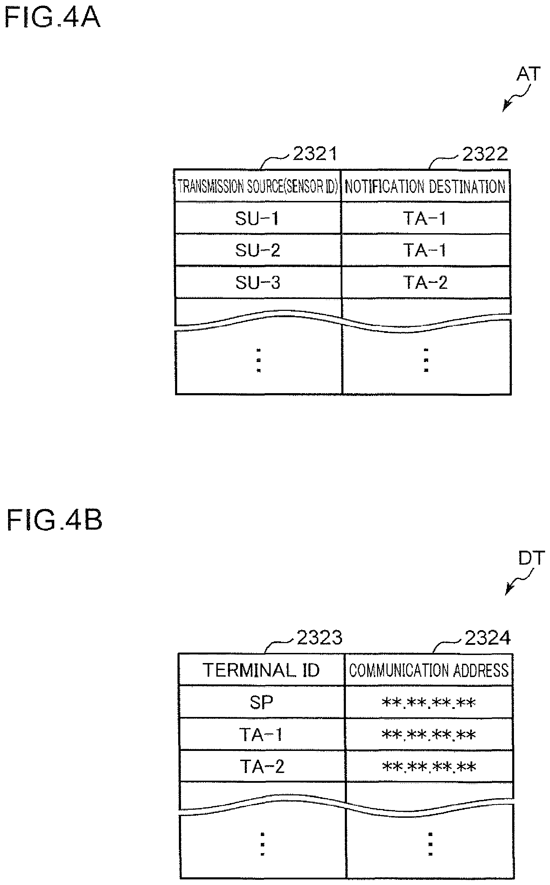

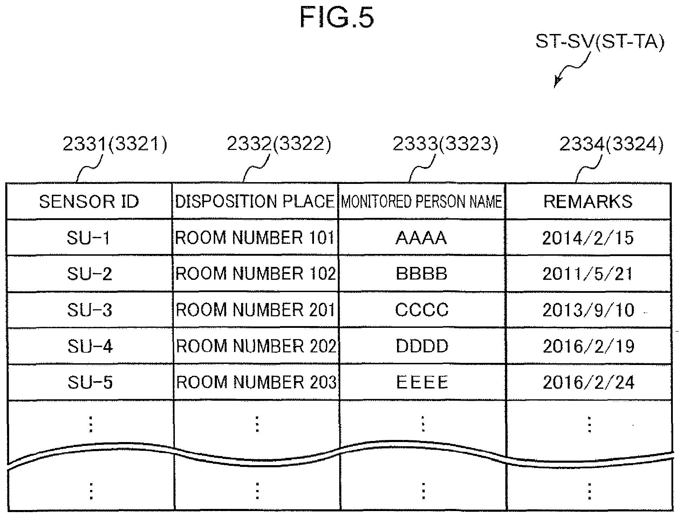

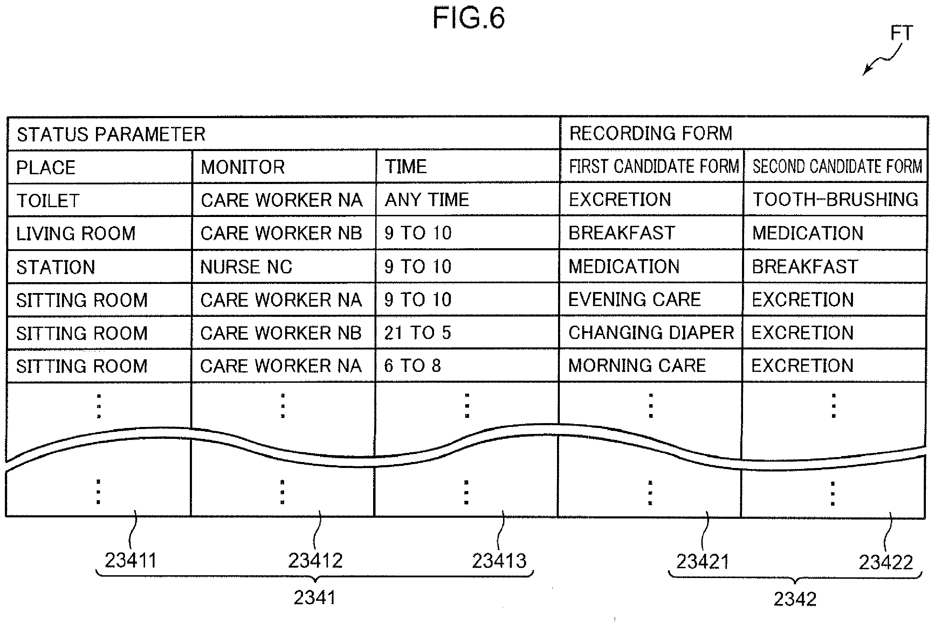

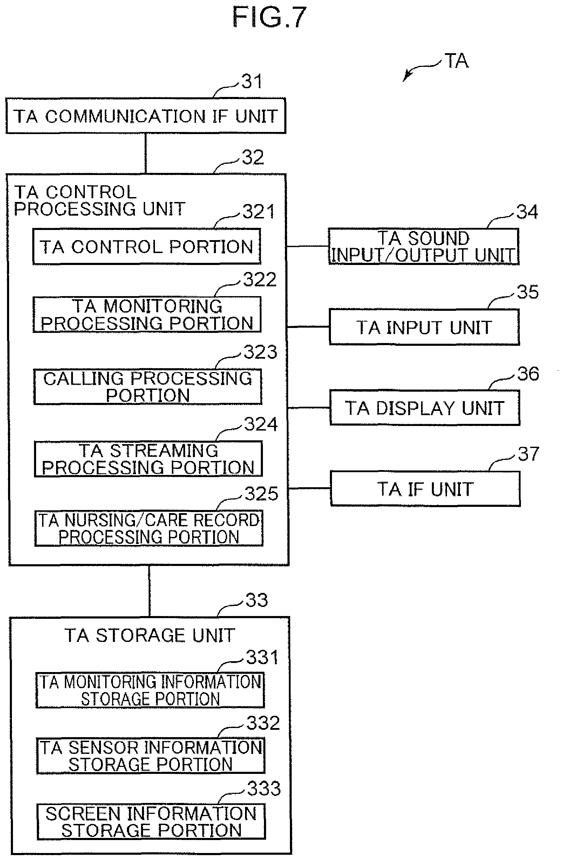

FIG. 1 is a diagram showing a configuration of the monitored person monitoring system in the embodiment. FIG. 2 is a diagram showing a configuration of the management server device of the monitored person monitoring system. FIG. 3 is a diagram showing a configuration of a server side monitoring information table stored in the management server device. FIG. 4 is a diagram showing configurations of inter-device information tables stored in the management server device. FIG. 4A shows a configuration of a notification destination correspondence relationship information table out of the inter-device information tables. FIG. 4B shows a configuration of a communication address correspondence relationship information table out of the inter-device information tables. FIG. 5 is a diagram showing a configuration of a server side sensor information table stored in the management server device. FIG. 6 is a diagram showing a configuration of a form information table stored in the management server device. FIG. 7 is a diagram showing a configuration of the mobile terminal device in the monitored person monitoring system.

More specifically, a monitored person monitoring system MS in the embodiment includes one or a plurality of sensor devices SU (SU-1 to SU-4), a management server device SV, a fixed terminal device SP, and one or a plurality of mobile terminal devices TA (TA-1, TA-2), a private branch exchange (PBX) CX as shown, for example, in FIG. 1, which are communicably connected by wire or wirelessly via a network (network, communication line) NW such as LAN (Local Area Network) or the like. The network NW may include a relay for relaying a communication signal, such as a repeater, a bridge, a router or the like. In the example shown in FIG. 1, the plurality of sensor devices SU-1 to SU-4, the management server device SV, the fixed terminal device SP, the plurality of mobile terminal devices TA-1 and TA-2, and the private branch exchange CX are communicably connected to each other by the LAN (e.g. IEEE802.11 standard LAN or the like) NW in which wired and wireless communication are mixedly present, the LAN NW including a hub (HUB) LS of an L2 switch and an access point AP. In more detail, the plurality of sensor devices SU-1 to SU-4, the management server device SV, the fixed terminal device SP, and the private branch exchange CX are connected to the hub LS, and the plurality of mobile terminal devices TA-1 and TA-2 are connected to the hub LS via the access point AP. Although the single hub LS and the single access point AP are illustrated in the example shown in FIG. 1, a plurality of hubs and access points may be provided. The network NW configures so-called intranet using an Internet protocol group including TCP (Transmission control protocol), IP (Internet protocol) and the like.

The monitored person monitoring system MS is disposed at an appropriate place according to the monitored person Ob. The monitored person (observed person) Ob is, for example, one who requires nursing due to sickness, injury or the like, one who requires care due to deterioration of physical ability or the like, one who lives alone, or the like. In particular, in view of enabling early detection and early coping, the monitored person Ob is preferably one who requires detection of a predetermined undesirable event, for example, abnormal state, occurring to the person. Therefore, the monitored person monitoring system MS is suitably disposed in a building such as a hospital, a welfare facility for the aged, and a house according to the type of the monitored person Ob. In the example shown in FIG. 1, the monitored person monitoring system MS is disposed in a building of a care facility equipped with a plurality of sitting rooms such as a plurality of sitting rooms RM where a plurality of monitored persons Ob live, and a nurses' station.

The sensor device SU is a device which has a communication function of communicating with other devices SV, SP, and TA via the network NW, and other functions, and detects a predetermined event related to the monitored person Ob to notify the detected event to the management server device SV. The predetermined event, which is preferably a predetermined event to be coped with, is for example, predetermined action set in advance for the monitored person Ob and nurse call (NC) in the present embodiment. More specifically, the sensor device SU is configured to include a communication interface circuit (e.g. LAN card or the like) for communicating with, for example, other devices SV, SP, and TA via the network NW, an image sensor which captures an image of the monitored person Ob to generate an image, an action detection processing circuit which detects predetermined action set in advance of the monitored person Ob as one example of the predetermined event on the basis of an output (image) of the image sensor and notifies the management server device SV of the detected action, a nurse call processing circuit which accepts a nurse call as another one example of the predetermined event and notifies the management server device SV of the nurse call, a calling processing circuit which conducts voice calling with the terminal device SP, TA or the like, an image transmission processing circuit which transmits an output (image (including still picture and moving picture)) of the image sensor to the predetermined other devices SV, SP, and TA, a control circuit which controls these circuits, and a peripheral circuit thereof.

The predetermined action in the present embodiment includes, for example, four actions, wake-up representing that the monitored person Ob wakes up, get-up representing that the monitored person Ob leaves bedding, fall representing that the monitored person Ob falls down from bedding, and tumble representing that the monitored person Ob tumbles. The action detection processing circuit detects a head of the monitored person Ob on the basis of, for example, an image (target image) captured by the image sensor, and detects wake-up, get-up, tumble, and fall of the monitored person Ob on the basis of a temporal change in a size of the detected head of the monitored person Ob. In more detail, there are first stored in advance a bedding BD locating region, a first threshold value Th1 for discrimination between a head size in a lying position within the bedding BD locating region and a head size in a sitting position, a second threshold value Th2 for discrimination of a head size in a standing position in the sitting room RM excluding the bedding BD locating region, and a third threshold value Th3 for discrimination of a head size in the lying position in the sitting room RM excluding the bedding BD locating region. And, the action detection processing circuit extracts a kinetic region as a region of a character of the monitored person Ob from a target image by, for example, background difference method or inter-frame difference method. The action detection processing circuit extracts a head region of the monitored person Ob from the extracted kinetic region by, for example, round or oval Hough transformation, or by pattern matching using, for example, a head model prepared in advance, or by a neural network learned for, for example, detecting a head. And, the action detection processing circuit detects wake-up, get-up, tumble, and fall from the extracted head position, and size. For example, the action detection processing circuit determines the action to be wake-up and detects the wake-up, in a case where the extracted head position is within the bedding BD locating region, and the extracted head size temporally changes from a size at the lying position to a size at the sitting position by using the first threshold value Th1. For example, the action detection processing circuit determines the action to be get-up and detects the get-up, in a case where the extracted head position is a result of a temporal change from inside the bedding BD locating region to the outside of the bedding locating region, and the extracted head size temporally changes from a certain size to a size at the standing position by using the second threshold value Th2. For example, the action detection processing circuit determines the action to be fall and detects the fall, in a case where the extracted head position temporally changes from inside the bedding BD locating region to the outside of the bedding BD locating region, and the extracted head size temporally changes from a certain size to the size at the lying position by using the third threshold value Th3. For example, the action detection processing circuit determines the action to be tumble and detects the tumble, in a case where the extracted head position is within the sitting room RM excluding the bedding BD locating region, and the extracted head size temporally changes from a certain size to the size at the lying position by using the third threshold value Th3.

The predetermined event is notified to the management server device SV from the sensor device SU by a first event notification communication signal. The first event notification communication signal contains a sensor ID of the own device and the event information indicative of contents of the event. The sensor ID (sensor identifier) is an identifier which specifies and identifies a sensor device SU. In the present embodiment, the event information is one or a plurality of events, i.e. wake-up, get-up, fall, tumble, and nurse call. The first event notification communication signal may contain an image captured by the image sensor. In particular, in a case where the event information is one of wake-up, get-up, fall, and tumble, it is preferable to contain an image used for the detection of the predetermined action (e.g. a last image in a case where the detection is conducted with a plurality of images). The image may be at least one of still picture and moving picture and in the present embodiment, first, still picture is notified and according to a user's request, moving picture is distributed as will be described later. Moving picture may be first distributed, or still picture and moving picture may be transmitted and then still picture and moving picture may be displayed in divisional screens on the terminal devices SP, TA.

FIG. 1 shows, as one example, four of first to fourth sensor devices SU-1 to SU-4, in which the first sensor device SU-1 is disposed in a sitting room RM-1 (not shown) of A called Ob-1, one of the monitored persons Ob, a second sensor device SU-2 is disposed in a second sitting room RM-2 (not shown) of B called Ob-2, one of the monitored persons Ob, the third sensor device SU-3 is disposed in a sitting room RM-3 (not shown) of C called Ob-3, one of the monitored persons Ob, and then, the fourth sensor device SU-4 is disposed in a sitting room RM-4 (not shown) of D called Ob-4, one of the monitored persons Ob.

The management server device SV is a device (central processing unit), which has a communication function of communicating with other devices SU, TA, SP via the network NW, and upon reception of a notification of the predetermined event from the sensor device SU, manages information related to monitoring of the monitored person Ob (monitoring information (in the present embodiment, e.g. the predetermined event (the kind of predetermined action detected by the sensor device SU or a nurse call accepted by the sensor device SU), an image of the monitored person Ob (still picture and moving picture), and time when the notification is received, etc.)), notifies (re-notifies, re-informs, re-transmits) the predetermined event to the predetermined terminal device SP, TA, provides a client with data according to a request of the client (the terminal device SP, TA, etc. in the present embodiment), and manages the monitored person monitoring system MS in its entirety. And, in the present embodiment, the management server device SV stores a plurality of recording forms according to the type of nursing/care, and upon reception of a request for a recording form from the terminal device SP, TA, returns a recording form based on a status parameter at the terminal device SP, TA as a transmission source of the request to the terminal device SP, TA as the transmission source. Upon receiving a nursing/care record in the recording form, the management server device SV stores the nursing/care record. The management server device SV like this includes a server side communication interface unit (SV communication IF unit) 21, a server side control processing unit (SV control processing unit) 22, and a server side storage unit (SV storage unit) 23 as shown, for example, in FIG. 2.

The SV communication IF unit 21 is a communication circuit connected to the SV control processing unit 22 to conduct communication according to control of the SV control processing unit 22. The SV communication IF unit 21 generates a communication signal containing data to be transferred which is input from the SV control processing unit 22 according to a communication protocol used in the network NW of the monitored person monitoring system MS, and transmits the generated communication signal to other devices SU, SP, TA via the network NW. The SV communication IF unit 21 receives the communication signal from the other devices SU, SP, TA via the network NW, extracts data from the received communication signal, converts the extracted data into data of a mode processable by the SV control processing unit 22, and outputs the converted data to the SV control processing unit 22. The SV communication IF unit 21 is configured to include the communication interface circuit conforming to, for example, IEEE802.11 standard or the like.

The SV storage unit 23 is a circuit connected to the SV control processing unit 22 to store various kinds of predetermined programs and various kinds of predetermined data under control of the SV control processing unit 22. The various kinds of predetermined programs include control processing programs such as an SV control program which controls each portion of the management server device SV according to a function of the portion, an SV monitoring processing program which executes predetermined information processing related to monitoring of the monitored person Ob, a form distribution processing program which returns, upon reception of a request for a recording form from the terminal device SP, TA, to the terminal device SP, TA as a transmission source, a recording form on the basis of a status parameter in the terminal device SP, TA as the transmission source, and an SV nursing/care record processing program which stores, upon reception of a nursing/care record in the recording form, the nursing/care record. The various kinds of predetermined data include data or the like necessary for executing each program, such as a server identifier (server ID) for specifying the management server device SV of own device to identify the management server device SV, the monitoring information of a monitored person Ob, inter-device information indicative of such information between the devices SU, SP, TA as a notification destination of the predetermined event, sensor information related to the sensor device SU, form information related to the recording form, nursing/care record information as information about a nursing/care record, and the like. The SV storage unit 23 includes, for example, a ROM (Read Only Memory) which is a non-volatile storage element, an EEPROM (Electrically Erasable Programmable Read Only Memory) as a rewritable non-volatile storage element, and the like. The SV storage unit 23 includes a RAM (Random Access Memory) or the like as a so-called working memory of the SV control processing unit 22, the memory storing data and the like generated during execution of the predetermined program. And, for storing these monitoring information, inter-device information, sensor information, form information, and nursing/care record information, the SV storage unit 23 functionally includes a server side monitoring information storage portion (SV monitoring information storage portion) 231, an inter-device information storage portion 232, a server side sensor information storage portion (SV sensor information storage portion) 233, a form information storage portion 234, and a nursing/care record information storage portion 235.

The SV monitoring information storage portion 231 stores monitoring information of the monitored person Ob transmitted/received between the respective devices SU, SP, and TA. More specifically, there are stored, so as to be correlated with each other in the SV monitoring information storage portion 231 as the monitoring information in the present embodiment, the sensor ID, the event information (the event information: wake-up, get-up, fall, tumble, and nurse call in the present embodiment), reception time, target image (still picture and moving picture), and presence/absence of coping on the basis of each piece of information contained in the communication signal such as the first event notification communication signal, or the like.

The monitoring information is stored in the SV monitoring information storage portion 231 in a table form in the present embodiment. A server side monitoring information table (SV monitoring information table) MT-SV, in which the monitoring information is registered, includes a sensor ID field 2311 in which a sensor ID contained in a communication signal received from each device SU, SP, TA is registered, an event field (event field) 2312 in which event information contained in the received communication signal is registered, a reception time field 2313 in which reception time of the received communication signal is registered, a still picture field 2314 in which the still picture contained in the received communication signal is registered, a moving picture field 2315 in which a communication address (e.g. IP address etc.) of a sensor device SU corresponding to the sensor ID contained in the received communication signal is registered as a live moving picture acquisition destination, and a handling field 2316 in which presence/absence of reception of handling the event information contained in the received communication signal is registered as shown, for example, in FIG. 3, and has a record for each received communication signal (for each event). In the still picture field 2314, for example, image data of still picture may be registered, and, for example, a file name of image data of still picture may be registered. In the handling field 2316, a flag (handling flag) is registered indicating whether the terminal device SP, TA accepts an intention ("handle") to cope (handling, treatment, measure) with the event information contained in the received communication signal or not as will be described later. For example, in the handling field 2316 of the present embodiment, a handling flag "1" is registered indicating that the terminal device SP, TA accepts an intention ("handle") to cope with the event information (the event information registered in the event field 2312) contained in the received communication signal, or a handling flag "0" is registered indicating that the terminal device SP, TA does not accept an intention ("handle") to cope with the event information contained in the received communication signal. By default, the handling flag "0" representing non-acceptance is registered in the handling field 2316. In a case where the first event notification communication signal contains detection time when the predetermined action is detected or nurse call reception time when the nurse call is received, the detection time or the nurse call reception time may be registered in place of reception time.

The inter-device information storage portion 232 in the present embodiment stores, as the inter-device information, a correspondence relationship (notification destination correspondence relationship) between a sensor ID as a transmission source and a terminal ID as an informing destination (re-informing destination), the relationship representing a notification destination (re-notification destination, re-informing destination, transmission destination) of the first event notification communication signal or the like transmitted from the sensor device SU, and a correspondence relationship (communication address correspondence relationship) between an ID (sensor ID, terminal ID) of each device SU, SP, TA and a communication address thereof, and the like. The terminal ID (terminal identifier) is an identifier which specifies the terminal device SP, TA to identify the terminal device SP, TA.

Each of the notification destination correspondence relationship and the communication address correspondence relationship is stored in a table form in the inter-device information storage portion 232 in the present embodiment. A notification destination correspondence relationship information table AT in which the notification destination correspondence relationship is registered has, for example, as shown in FIG. 4A, a transmission source field 2321 in which a sensor ID of the sensor device SU as a transmission source is registered, and a notification destination field 2322 in which a terminal ID of the terminal device SP, TA as a transmission destination is registered, the transmission destination being a destination to which a communication signal notified from the sensor device SU corresponding to the sensor ID registered in the transmission source field 2321 is transmitted, and has a record for each sensor ID (the sensor device SU). A communication address correspondence relationship information table DT in which the communication address correspondence relationship is registered has, for example, as shown in FIG. 4B, a terminal ID field 2323 in which a terminal ID of the terminal device SP, TA is registered, and a communication address field 2324 in which a communication address of the terminal device SP, TA corresponding to the terminal ID registered in the terminal ID field 2323 is registered, and has a record for each terminal ID (terminal device SP, TA).

The sensor ID, the server ID, and the terminal ID each may be a serial number composed of, for example, a predetermined symbol sequence, or the like, or may be, for example, a communication address (in this case, the communication address correspondence relationship can be omitted). Additionally, as a notification destination correspondence relationship, a plurality of sensor devices SU can be correlated with one terminal device SP, TA.

The SV sensor information storage portion 233 stores the sensor information. In the present embodiment, the sensor information is information related to the sensor device SU, the information correlating a sensor ID of the sensor device SU and a monitored person name of the monitored person Ob with each other.

In the present embodiment, such sensor information is stored in a table form in the SV sensor information storage portion 233. More specifically, a server side sensor information table (SV sensor information table) ST-SV in which sensor information is registered has, for example, as shown in FIG. 5, a sensor ID field 2331 in which a sensor ID is registered, a disposition place field 2332 in which a disposition place of the sensor device SU having the sensor ID registered in the sensor ID field 2331 is registered, a monitored person name field 2333 in which a monitored person name of a monitored person Ob (i.e. a monitored person Ob at a disposition place of the sensor device SU having the sensor ID registered in the sensor ID field 2331) is registered, the monitored person Ob being monitored by the sensor device SU having the sensor ID registered in the sensor ID field 2331, and a remarks field 2334 in which remarks are registered about the sensor device SU having the sensor ID registered in the sensor ID field 2331, about a disposition place of the sensor device, and about the monitored person Ob, and has a record for each sensor ID (i.e. the sensor device SU).

The form information storage portion 234 stores the form information. The form information is information related to a recording form used for inputting contents of nursing/care including at least one of nursing and care as a nursing/care record, and a plurality of the recording forms are prepared in advance according to the type of nursing/care in a predetermined format (format, input form) defined in advance.

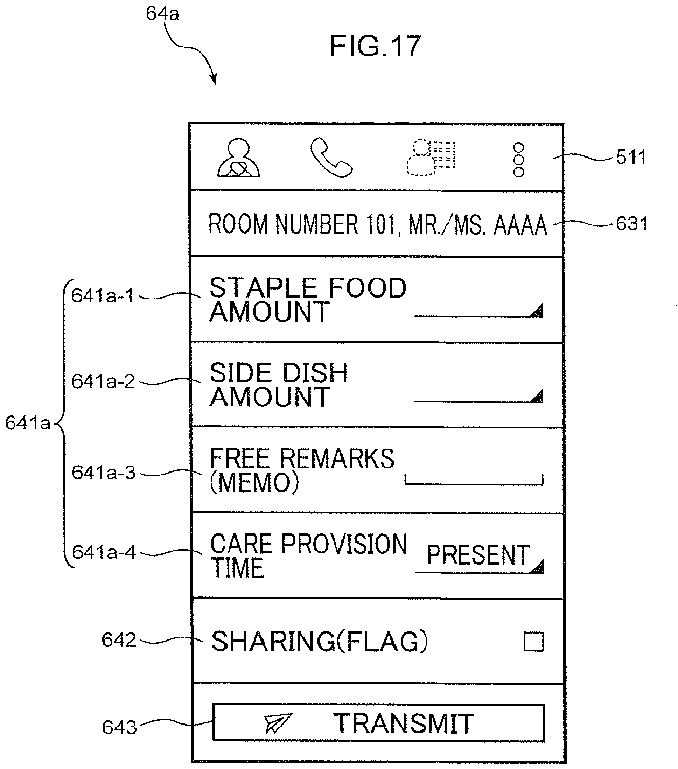

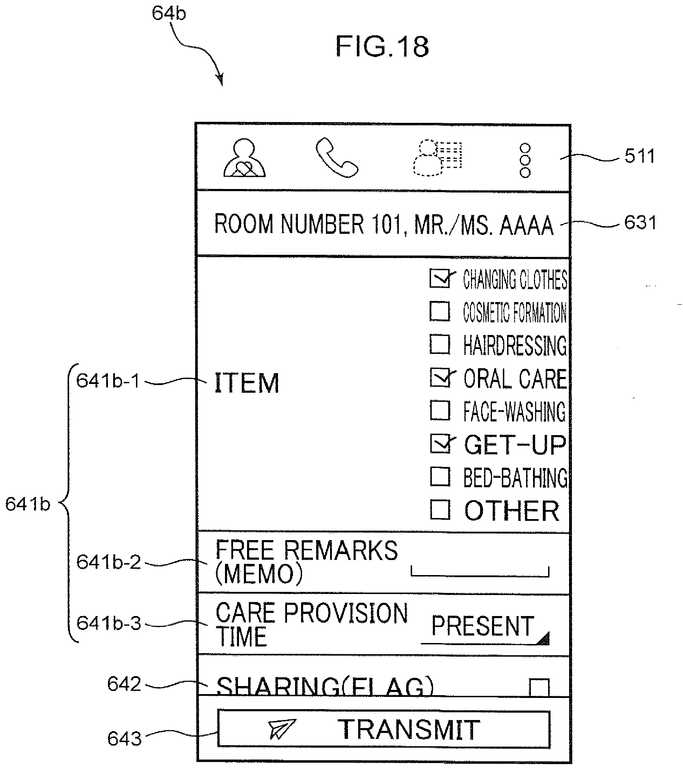

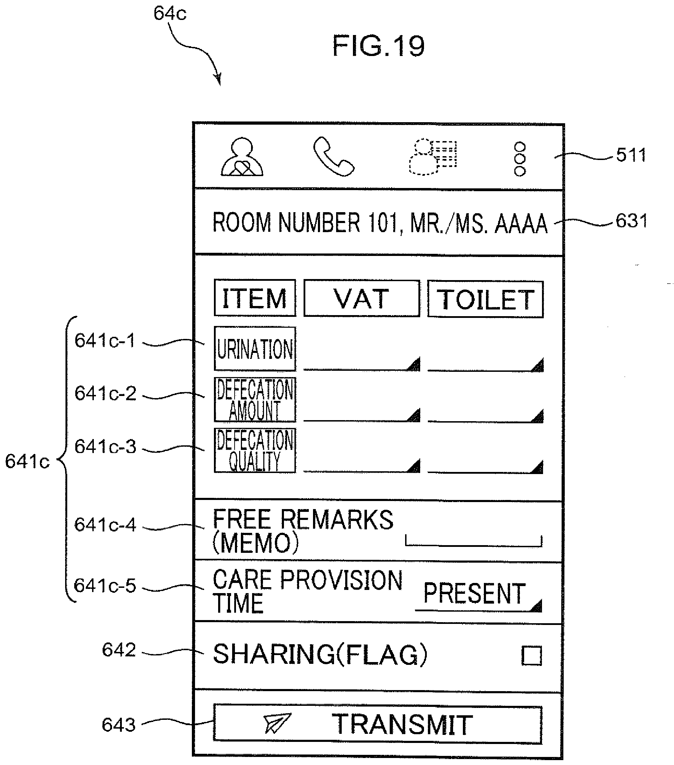

Various types of nursing/care are provided according to practical work of nursing, practical work of care, or the like. Among the types of nursing/care are, for example, care having periodicity, care depending on a place, etc. More specifically, among care having periodicity are care conducted daily such as "morning care" (change of clothes, face-washing, attachment of dentures, etc.), meal care (breakfast care, lunch care, evening meal car), eating between meals care, taking-medicine care, tooth-brushing care, hydration care, vital check, evening care (change of clothes, tooth-brushing, detachment of dentures, bed change, etc.), patrol, posture change, etc., and care conducted on a certain day of the week or on certain date, such as bathing care, rehabilitation, recreation, house call, body bed-bathing, measuring of weight, etc. Care depending on a place includes excretion care and the like. The foregoing nursing/care depends on a place such as a living room, a sitting room RM, a toilet, a bathroom, a nurses' station or the like, or depends on, for example, a nurse, a care worker or the like, or a monitor of a person in charge of the nursing/care or the like. The recording forms are prepared in advance according to such contents of nursing/care and include, for example, a recording form for morning care, a recording form for meal care, a recording form for eating between meals care, a recording form for taking-medicine care, a recording form for tooth-brushing care, a recording form for hydration care, and the like. One example of the cares is shown in FIG. 17 to FIG. 19 which will be described later. FIG. 17 shows one example of a recording form for meal care indicated on a screen displayed in the mobile terminal device TA. FIG. 18 shows one example of a recording form for morning care indicated on a screen displayed in the mobile terminal device TA. FIG. 19 shows one example of a recording form for excretion care indicated on a screen displayed in the mobile terminal device TA.

Such form information is stored in the form information storage portion 234 so as to be correlated with any of the plurality of recording forms different from each other according to types of nursing/care with respect to a predetermined status parameter. The status parameter is a parameter indicative of a predetermined status in a transmission source requesting a recording form. The recording form in many cases depends on a position (place), a monitor (user), time, and the like as described above. For example, a recording form for excretion care is often used near a toilet, and therefore depends on a position (place). In a case, for example, where a person in charge of a monitored person A is a monitor NA, a recording form for a monitored person depends on a monitor. Additionally, for example, a recording form for meal care, which is often used near meal time, depends on time. Therefore, in the present embodiment, the status parameter is at least one of, for example, position information indicative of a position of the transmission source, monitor information indicative of a monitor handling the terminal device SP, TA as the transmission source, and time of request for a recording form. In the present embodiment, since a request for the recording form is received through a communication signal (form request communication signal) which contains a status parameter indicative of a predetermined status of a transmission source and an instruction (command) requesting a recording form, time of a request for a recording form is reception time of the form request communication signal. In the present embodiment, the status parameter is configured to include the position information, the monitor information, and the reception time. Such form information in the present embodiment is stored, for example, in a table form in the form information storage portion 234. More specifically, a form information table FT in which form information is registered has, for example, a status parameter field 2341 in which a status parameter is registered, and a recording form field 2342 in which a recording form corresponding to a status parameter registered in the status parameter field 2341 is registered as shown in FIG. 6, and has a record for each status parameter. In the present embodiment, the status parameter is configured to include position information, monitor information, and reception time as described above, and therefore, the status parameter field 2341 has a place sub-field 23411 in which the position information is registered, a monitor sub-field 23412 in which the monitor information is registered, and a time sub-field 23413 in which the reception time is registered. Additionally, a plurality of candidates is prepared as a recording form corresponding to a status parameter. In the present embodiment, for example, two candidates are prepared, and therefore the recording form field 2342 has a first candidate form sub-field 23421 in which a first candidate recording form is registered, and a second candidate form sub-field 23422 in which a second candidate recording form is registered. Although in the first and second candidate form sub-fields 23421 and 23422, information of a recording form itself may be registered, a file name (e.g. recording form name etc.) in an electronic file in a recording form is registered in the present embodiment. Electronic file in the recording form is described with markup languages such as HTML (Hyper Text Markup Language), XML (Extensible Markup Language), and the like, and is stored in the form information storage portion 234.

The nursing/care record information storage portion 235 stores the nursing/care record information. A monitored person name as a target person for nursing/care, and contents of the nursing/care record are recorded as the nursing/care record information in the nursing/care record information storage portion 235 so as to be correlated with each other.

The SV control processing unit 22 is a circuit which controls each portion of the management server device SV according to a function of the portion, and upon reception of a notification of the predetermined event from the sensor device SU, manages monitoring information related to monitoring of the monitored person Ob, notifies (re-notifies, re-informs, transmits) the predetermined event to a predetermined terminal device SP, TA, provides a client with data according to the client's request, and manages the monitored person monitoring system MS in its entirety. And, in the present embodiment, upon reception of a request for a recording form from the terminal device SP, TA, the SV control processing unit 22 returns, to the terminal device SP, TA as a transmission source, a recording form based on a status parameter in the terminal device SP, TA as the transmission source. Upon reception of a nursing/care record in a recording form, the SV control processing unit 22 stores the nursing/care record. The SV control processing unit 22 is configured to include, for example, a CPU (Central Processing Unit) and peripheral circuits thereof. As a result of execution of the control processing program, the SV control processing unit 22 functionally includes a server side control portion (SV control portion) 221, a server side monitoring processing portion (SV monitoring processing portion) 222, a form distribution processing portion 223, and a server side nursing/care record processing portion (SV nursing/care record processing portion) 224.

The SV control portion 221 controls each portion of the management server device SV according to a function of the portion, and is in charge of entire control of the management server device SV.

Upon reception of a notification of the predetermined event from the sensor device SU, the SV monitoring processing portion 222 manages monitoring information related to monitoring of the monitored person Ob and notifies the predetermined event to the predetermined terminal device SP, TA. More specifically, upon reception of the first event notification communication signal from the sensor device SU, the SV monitoring processing portion 222 stores (records), in the SV monitoring information storage portion 231, monitoring information related to monitoring of the monitored person Ob contained in the received first event notification communication signal. And, the SV monitoring processing portion 222 selects (searches) an informing destination (re-informing destination, transfer destination, transmission destination) corresponding to the sensor device SU having transmitted the received first event notification communication signal from the notification destination correspondence relationship stored in the inter-device information storage portion 232, and transmits a second event notification communication signal to the selected terminal device SP, TA. This selection (search processing) is conducted on the basis of a sensor ID corresponding to the sensor device SU having transmitted the received first event notification communication signal. In a case where the event information contained in the first event notification communication signal is the predetermined action (one or a plurality of wake-up, get-up, fall, and tumble), the second event notification communication signal contains a sensor ID, event information, and a target image contained in the first event notification communication signal, and as a download destination of moving picture, contains a communication address corresponding to the sensor device SU having the sensor ID contained in the first event notification communication signal. The communication address is selected (searched) from the communication address correspondence relationship on the basis of the sensor ID corresponding to the sensor device SU having transmitted the received first event notification communication signal. In a case where the event information contained in the first event notification communication signal is the nurse call, the sensor ID and the event information (nurse call) contained in the first event notification communication signal are contained in the second event notification communication signal.

Upon reception of a request for a recording form from the terminal device SP, TA, the form distribution processing portion 223 returns, to the terminal device SP, TA as a transmission source of the request, a recording form based on a status parameter in the terminal device SP, TA as the transmission source. And, in the present embodiment, the form distribution processing portion 223 counts time and date. More specifically, when receiving a communication signal (form request communication signal (first form request communication signal)) at the SV communication IF unit 21, the signal containing a status parameter indicative of a predetermined status in a transmission source and an instruction (first instruction) requesting a recording form, the form distribution processing portion 223 selects a recording form from among a plurality of recording forms stored in the form information storage portion 234 on the basis of the status parameter contained in the received form request communication signal, and transmits a communication signal (form return communication signal (first form return communication signal)) containing the selected recording form to the transmission source by means of the SV communication IF unit 21. And, upon reception of a communication signal (second form request communication signal) at the SV communication IF unit 21 from the transmission source to which the first form return communication signal has been transmitted, the communication signal containing a second instruction requesting a second recording form different from the recording form contained in the first form return communication signal, the form distribution processing portion 223 selects a second recording form different from the recording form selected for the first form return communication signal from among the plurality of recording forms stored in the form information storage portion 234 on the basis of the status parameter contained in the first form request communication signal received from the transmission source, the first form request communication signal being derived from transmission of the first form return communication signal, and transmits a communication signal (second form return communication signal) containing the selected second recording form to the transmission source by means of the SV communication IF unit 21. In more detail, upon reception of a form request communication signal (first form request communication signal) from the terminal device SP, TA at the SV communication IF unit 21, the form distribution processing portion 223 selects (searches) a record with the status parameter field 2341 in which a status parameter contained in the received form request communication signal is registered from the form information table FT stored in the form information storage portion 234, extracts a recording form (a file name of an electronic file in a recording form in the present embodiment) registered in the first candidate form sub-field 23421 in the recording form field 2342 of the selected record, and transmits a form return communication signal (first form return communication signal) containing the extracted recording form (an electronic file in a recording form corresponding to the file name in the present embodiment) to the transmission source by means of the SV communication IF unit 21. And, upon reception of the second form request communication signal at the SV communication IF unit 21 from the transmission source to which the first form return communication signal has been transmitted, the form distribution processing portion 223 selects (searches), from the form information table FT stored in the form information storage portion 234, a record with the status parameter field 2341 in which the status parameter contained in the first form request communication signal received from the transmission source is registered, the first form request communication signal being derived from transmission of the first form return communication signal, extracts a recording form (a file name of an electronic file in a recording form in the present embodiment) registered in the second candidate form sub-field 23422 in the recording form field 2342 of the selected record, and transmits a second form return communication signal containing the extracted recording form (an electronic file in a recording form corresponding to the file name in the present embodiment) to the transmission source by means of the SV communication IF unit 21.

Upon reception of a nursing/care record in a recording form from the terminal device SP, TA, the SV nursing/care record processing portion 224 stores the nursing/care record in the nursing/care record information storage portion 235.

The management server device SV may further include a server side input unit (SV input unit) 24 connected to the SV control processing unit 22 for inputting, for example, various kinds of commands, various kinds of data, and the like, a server side output unit (the SV output unit) 25 for outputting various kinds of commands and various kinds of data input by the SV input unit 24 and monitoring information related to monitoring of the monitored person Ob, and a server side interface unit (SV IF unit) 26 for conducting data input/output to/from an external apparatus, and the like as required as indicated by dashed lines in FIG. 2.

Such a management server device SV can be configured by, for example, a computer with a communication function.

The fixed terminal device SP is an apparatus having a communication function of communicating with other device SU, SV, TA via the network NW, a display function of displaying predetermined information, an input function of inputting predetermined instruction and data, and other function, and functioning as a user interface (UI) of the monitored person monitoring system MS by inputting predetermined instruction and data to be applied to the management server device SV or the mobile terminal device TA, displaying monitoring information obtained from the sensor device SU, and the like. Such a fixed terminal device SP can be configured by, for example, a computer with a communication function. While the fixed terminal device SP as one example of the terminal device operates in the same manner as in the mobile terminal device TA, description will be made of the mobile terminal device TA as another example of the terminal device in the present specification.

The mobile terminal device TA is an apparatus, which has a communication function of communicating with other device SV, SP, SU via the network NW, a display function of displaying predetermined information, an input function of inputting predetermined instruction and data, a calling function of conducting voice calling, and other function, which inputs predetermined instruction and data to be applied to the management server device SV and the sensor device SU, displays the monitoring information obtained from the sensor device SU by a notification from the management server device SV, and conducts, with the sensor device SU, response to a nurse call, talking through voice calling, and the like. The mobile terminal device TA like this includes, for example, a terminal side communication interface unit (TA communication IF unit) 31, a terminal side control processing unit (TA control processing unit) 32, a terminal side storage unit (TA storage unit) 33, a terminal side sound input/output unit (TA sound input/output unit) 34, a terminal side input unit (TA input unit) 35, a terminal side display unit (TA display unit) 36, and a terminal side interface unit (TAIF unit) 37 in the present embodiment as shown in FIG. 7.

The TA sound input/output unit 34 is a circuit connected to the TA control processing unit 32 to acquire external sound and input the same to the mobile terminal device TA, the circuit generating and outputting an electric signal indicative of sound according to control of the TA control processing unit 32. The TA sound input/output unit 34 is configured to include, for example, a speaker or the like which converts an electric signal of sound (sound data) into a mechanical oscillation signal of sound (audio signal), and a microphone or the like which converts a mechanical oscillation signal of sound in an audible range into an electric signal. The TA sound input/output unit 34 outputs an electric signal indicative of external sound to the TA control processing unit 32, and also converts an electric signal input from the TA control processing unit 32 into a mechanical oscillation signal of sound and outputs the converted signal.

The TA input unit 35 is a circuit connected to the TA control processing unit 32 to, for example, accept predetermined operation and input the same to the mobile terminal device TA, and is, for example, a plurality of input switches each assigned a predetermined function. The predetermined operation includes various kinds of operation necessary for monitoring, such as, for example, input operation of an ID for log-in, requesting operation for voice calling and ending operation for the same, requesting operation for live moving picture and ending operation for the same, input operation of an intension ("handle") to execute handling (coping with, dealing with) such as life-saving, nursing, care and assistance, etc. of a monitored person Ob related to the notified event, input operation of a nursing/care record in a predetermined recording form, and the like. The TA display unit 36 is a circuit connected to the TA control processing unit 32 to display, under control of the TA control processing unit 32, predetermined operation contents input from the TA input unit 35, the monitoring information (e.g. the predetermined event (the type of predetermined action detected by the sensor device SU and a nurse call accepted by the sensor device SU), images (still picture and moving picture) of a monitored person Ob, and time when the notification is received, etc.) related to monitoring of the monitored person Ob monitored by the monitored person monitoring system MS, and a recording form used for inputting a nursing/care record, the circuit being a display device such as an LCD (liquid crystal display), an organic EL display, or the like. And, in the present embodiment, the TA input unit 35 and the TA display unit 36 configure a touch panel. In this case, the TA input unit 35 is a position input device which detects and inputs an operation position such as a resistive film method, an electrostatic capacitance method, or the like. In the touch panel, the position input device is provided on a display surface of the TA display unit 36, on which one or a plurality of candidates as input contents that can be input to the TA display unit 36 are displayed, so that when a user (monitor) such as a nurse, a care worker, or the like, for example, touches a display position in which input contents to be input are displayed, the position input device detects the touched position, and display contents displayed at the detected position are input to the mobile terminal device TA as operation input contents of the user.

The TA IF unit 37 is a circuit which is connected to the TA control processing unit 32 to conduct data input/output with an external apparatus under control of the TA control processing unit 32, the circuit being, for example, an interface circuit conforming to Bluetooth (registered trademark) standard, an interface circuit conducting infrared communication conforming to IrDA standard, an interface circuit conforming to USB standard, and the like.

The TA communication IF unit 31 is a communication circuit connected to the TA control processing unit 32 to conduct communication under control of the TA control processing unit 32 similarly to the SV communication IF unit 21. The TA communication IF unit 31 is configured to include a communication interface circuit conforming to, for example, IEEE802.11 standard.

The TA storage unit 33 is a circuit connected to the TA control processing unit 32 to store various kinds of predetermined programs and various kinds of predetermined data under control of the TA control processing unit 32. The various kinds of predetermined programs include control processing programs such as a TA control program for controlling each portion of the mobile terminal device TA according to a function of the portion, a TA monitoring processing program for executing predetermined information processing related to monitoring of the monitored person Ob, a calling processing program for conducing voice calling with the sensor device SU by using the TA sound input/output unit 34, etc., a TA streaming processing program for receiving distribution of moving picture from the sensor device SU to display the distributed moving picture on the TA display unit 36 by streaming reproduction, a TA nursing/care record processing program for executing input processing of a nursing/care record in a predetermined recording form, and the like. The various kinds of predetermined data include data etc. necessary for executing each program, such as a terminal ID of own device, the monitoring information of a monitored person Ob, sensor information related to the sensor device SU, and screen information displayed on the TA display unit 36. The TA storage unit 33 includes, for example, a ROM, an EEPROM, etc. The TA storage unit 33 includes a RAM or the like as a so-called working memory of the TA control processing unit 32, the memory storing data and the like generated during execution of the predetermined program. The TA storage unit 33 functionally includes, for storing these monitoring information, sensor information, and screen information, a terminal side monitoring information storage portion (TA monitoring information storage portion) 331, a terminal side sensor information storage portion (TA sensor information storage portion) 332, and a screen information storage portion 333.

The TA monitoring information storage portion 331 stores the monitoring information. In the present embodiment, the TA monitoring information storage portion 331 stores, as the monitoring information, a sensor ID and event information (event information; wake-up, get-up, fall, and tumble and nurse call in the present embodiment), and a communication address of the sensor device SU as a download destination of image and moving picture contained in a second event notification communication signal received from the management server device SV, and reception time of the second event notification communication signal and presence/absence of coping with the event, and the like, so as to be correlated with each other. More specifically, the TA monitoring information storage portion 331 stores the monitoring information in a terminal side monitoring information table (TA monitoring information table) MT-TA similar to the SV monitoring information table MT-SV as shown in FIG. 3.

The TA sensor information storage portion 332 stores the sensor information. The TA sensor information storage portion 332 stores a sensor ID, a disposition place, a monitored person name, and remarks so as to be correlated with each other. More specifically, the TA sensor information storage portion 332 stores the sensor information in a terminal side sensor information table (TA sensor information table) ST-TA similar to the SV sensor information table ST-SV as shown in FIG. 5.

The screen information storage portion 333 stores the screen information. The screen information storage portion 333 stores in advance electronic files used relatively frequently of a standby screen 51, a monitoring information screen 52, a nurse call reception screen 53 which will be described later, and the like, and also stores electronic files of a predetermined recording form screen 64 received from the management server device SV, and the like.

The TA control processing unit 32 is a circuit which controls each portion of the mobile terminal device TA according to a function of the portion, receives and displays the monitoring information of a monitored person Ob, and performs a response to a nurse call and talking. And, in the present embodiment, the TA control processing unit 32 executes input processing of a nursing/care record in a predetermined recording form. The TA control processing unit 32 is configured to include, for example, a CPU and peripheral circuits thereof. As a result of execution of the control processing program, the TA control processing unit 32 functionally includes a terminal side control portion (TA control portion) 321, a terminal side monitoring processing portion (TA monitoring processing portion) 322, a calling processing portion 323, a terminal side streaming processing portion (TA streaming processing portion) 324, and a terminal side nursing/care record processing portion (TA nursing/care record processing portion) 325.

The TA control portion 321 controls each portion of the mobile terminal device TA according to a function of the portion and is in charge of entire control of the mobile terminal device TA.

The TA monitoring processing portion 322 executes predetermined information processing related to monitoring of the monitored person Ob. More specifically, upon reception of a second event notification communication signal transmitted by the management server device SV derived from a first event notification communication signal transmitted by the sensor device SU, the TA monitoring processing portion 322 stores (records) monitoring information of the monitored person Ob in the TA monitoring information storage portion 331 on the basis of each piece of information (each piece of data) contained in the received second event notification communication signal. The TA monitoring processing portion 322 displays, on the TA display unit 36, a screen according to each piece of information contained in the received second event notification communication signal. And, when accepting predetermined input operation from the TA input unit 35, the TA monitoring processing portion 322 executes predetermined processing according to the input operation.

The calling processing portion 323 conducts voice calling with the sensor device SU by means of the TA sound input/output unit 34, etc. More specifically, the calling processing portion 323 conducts voice calling, for example, by means of VoIP (Voice over Internet Protocol) using the TA sound input/output unit 34 or the like, with the sensor device SU as a transmission source which has transmitted, to the management server device SV, a first event notification communication signal which caused transmission of a second event notification communication signal.

The TA streaming processing portion 324 receives distribution of moving picture (e.g. live moving picture) from the sensor device SU to display the distributed moving picture on the TA display unit 36 by streaming reproduction.

The TA nursing/care record processing portion 325 executes input processing of a nursing/care record in a predetermined recording form. More specifically, the TA nursing/care record processing portion 325 executes, in two of first and second modes, input processing of a nursing/care record. In the first mode, the TA nursing/care record processing portion 325 sequentially executes each processing, i.e. target person selecting processing of selecting a target person of a nursing/care record, form selection processing of selecting a recording form, form request display processing of requesting the management server device SV for a recording form to display the recording form on the TA display unit 36, and recording processing of accepting input of a nursing/care record in a recording form at the TA input unit 35 to transmit the record to the management server device SV. In the second mode, the TA nursing/care record processing portion 325 sequentially executes, upon accepting a request for a recording form at the TA input unit 35, each processing, i.e. acquisition processing of acquiring a status parameter (own device position information in the present embodiment) of own device, first form request display processing of transmitting, to the management server device SV, a first form request communication signal containing a terminal ID, a status parameter of own device, and an instruction (first instruction) requesting a recording form and displaying a recording form (a first candidate recording form) contained in the returned first form return communication signal on the TA display unit 36, second form request display processing of transmitting, to the management server device SV, as required, a second form request communication signal containing own device terminal ID, and a second instruction requesting a second recording form different from a recording form contained in the first form return communication signal and displaying a recording form (second candidate recording form) contained in the returned second form return communication signal on the TA display unit 36, and recording processing of accepting, at the TA input unit 35, input of a nursing/care record in a first candidate recording form or a second candidate recording form and transmitting the accepted record to the management server device SV.

The mobile terminal device TA like this can be configured by a portable communication terminal device such as a so-called tablet computer, a smartphone, or a mobile phone.

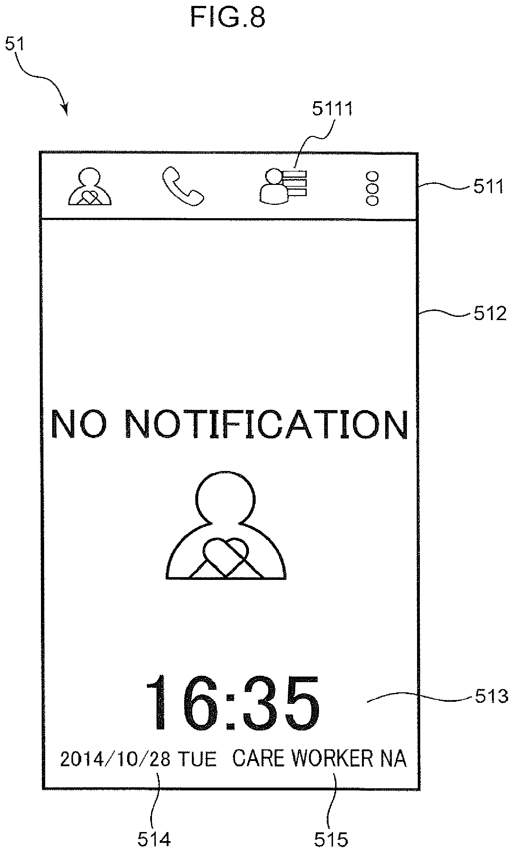

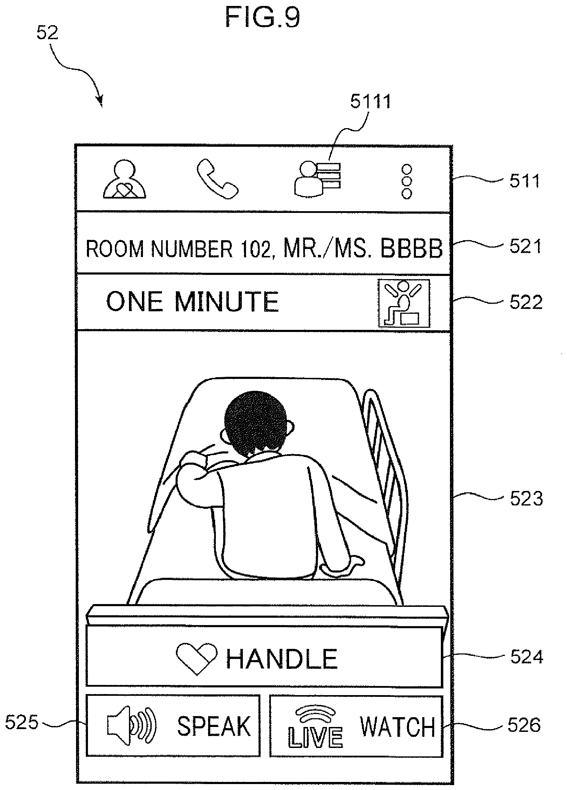

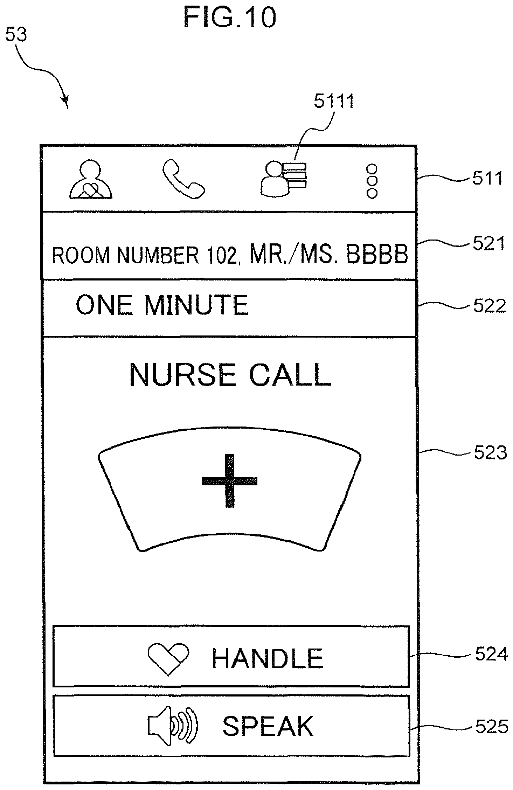

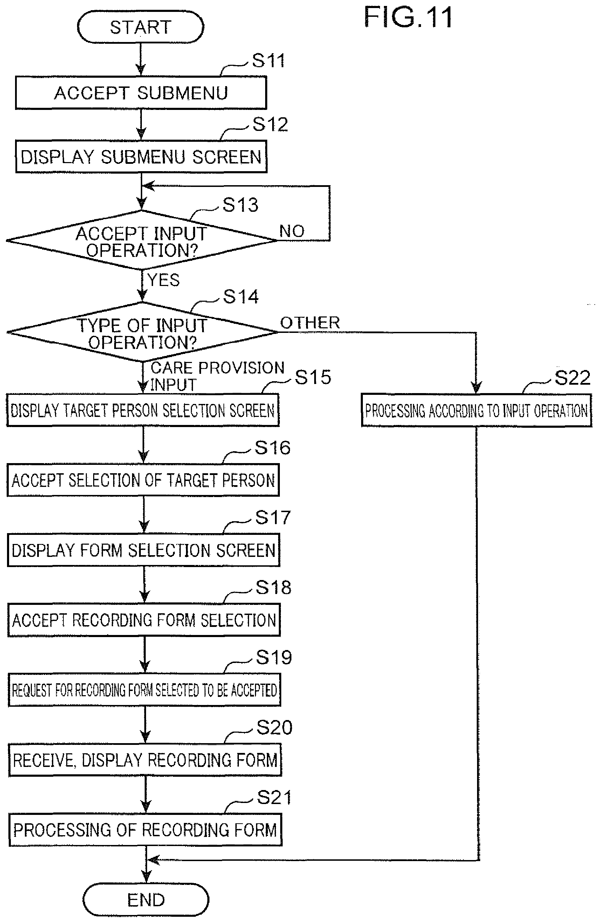

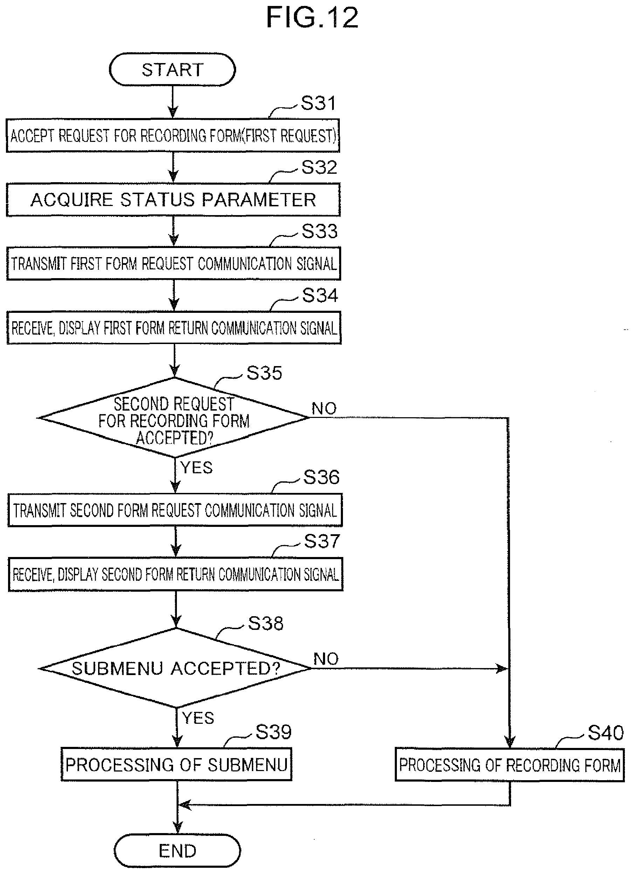

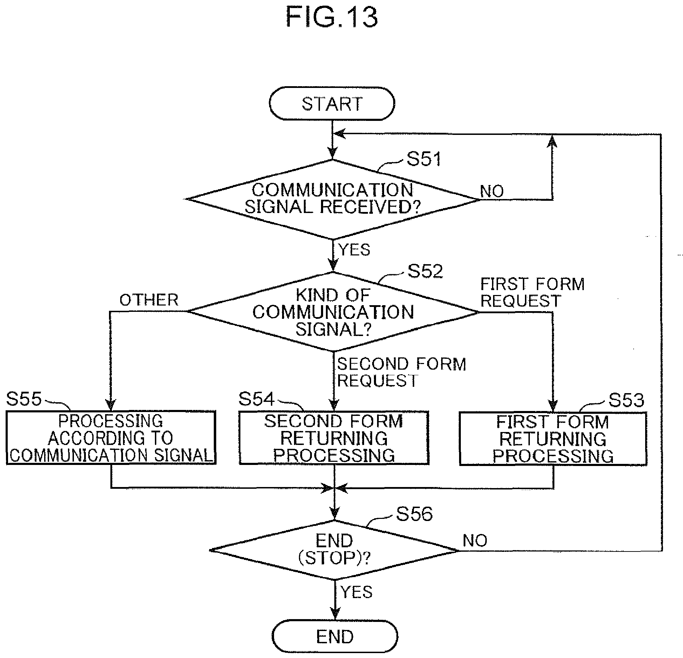

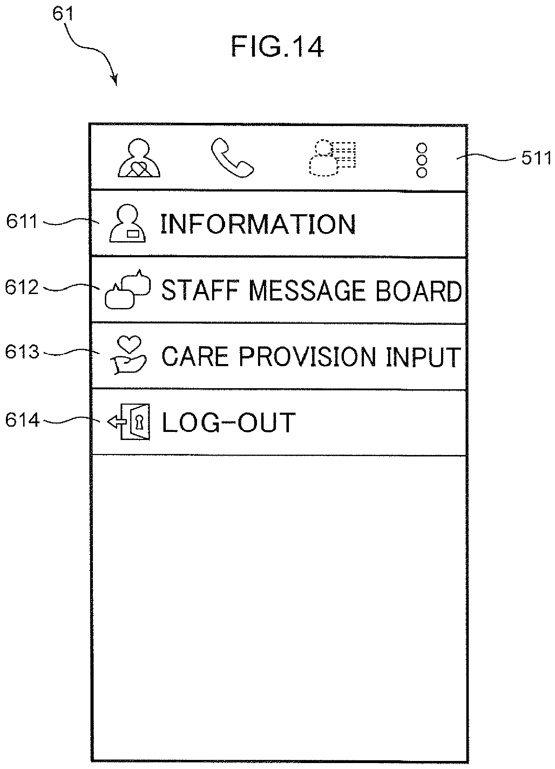

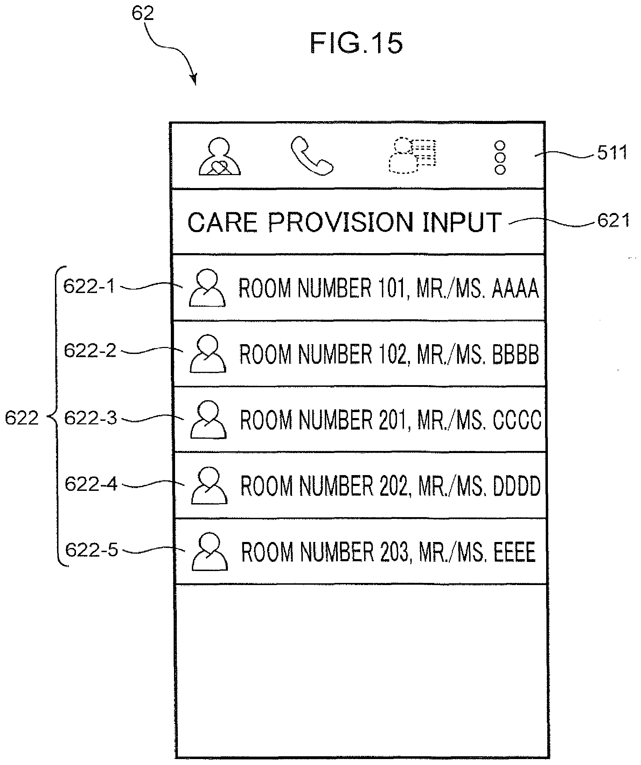

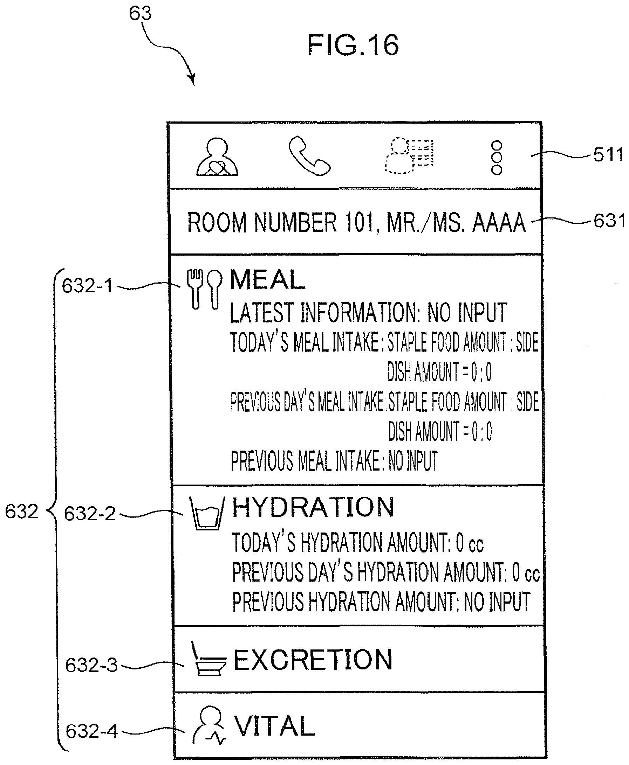

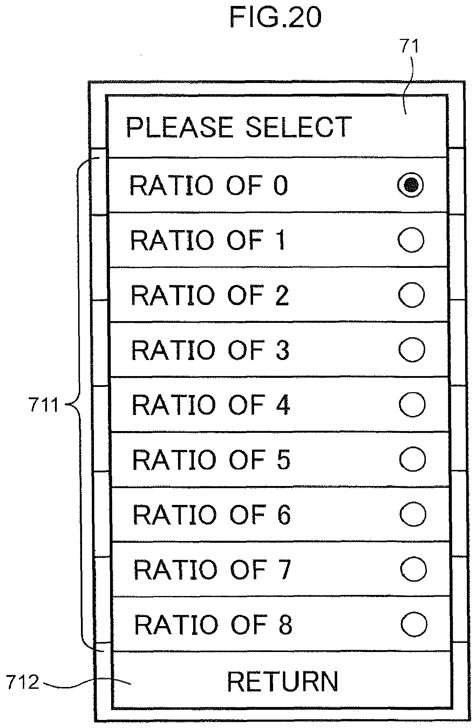

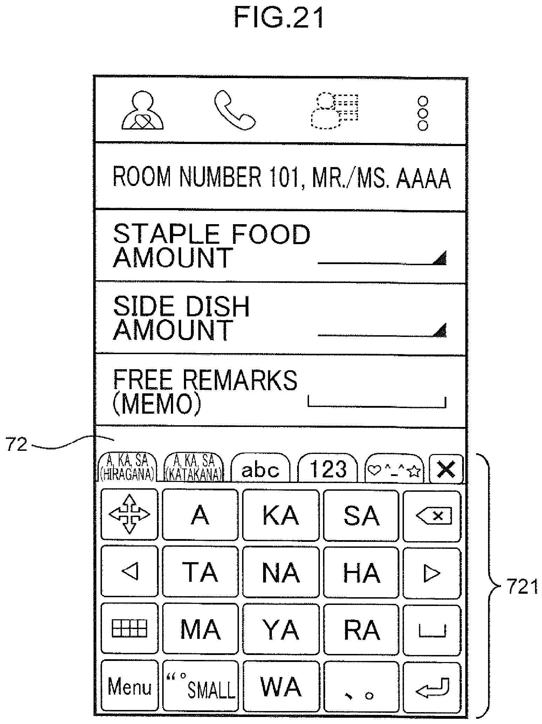

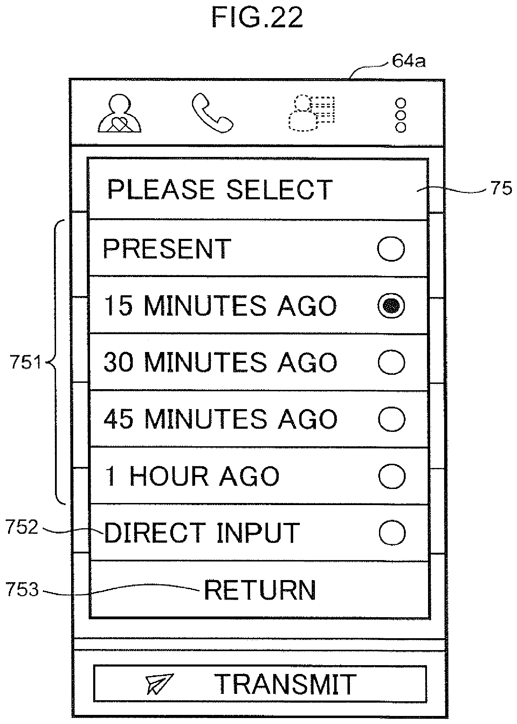

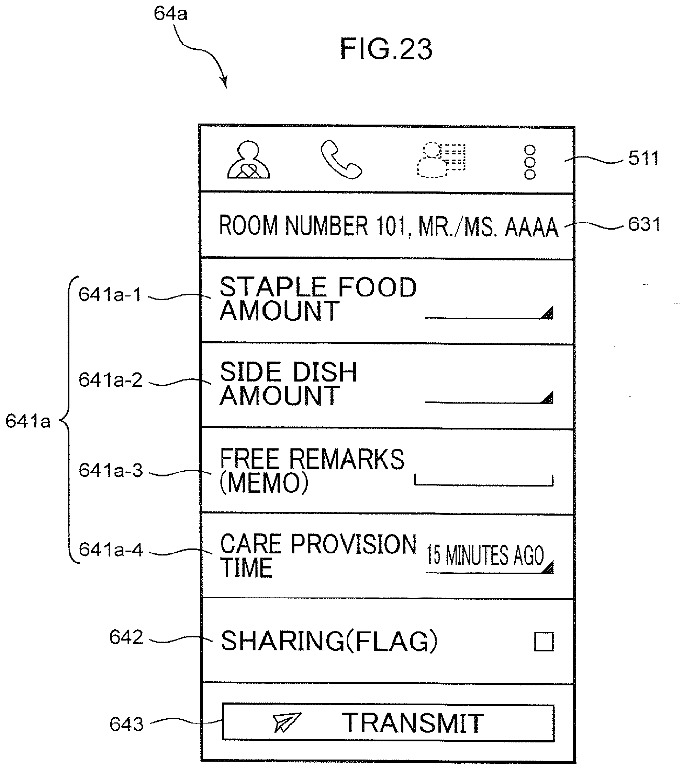

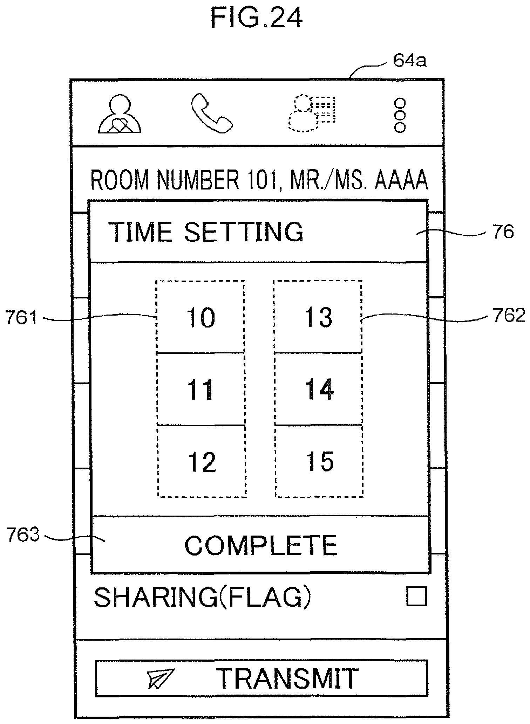

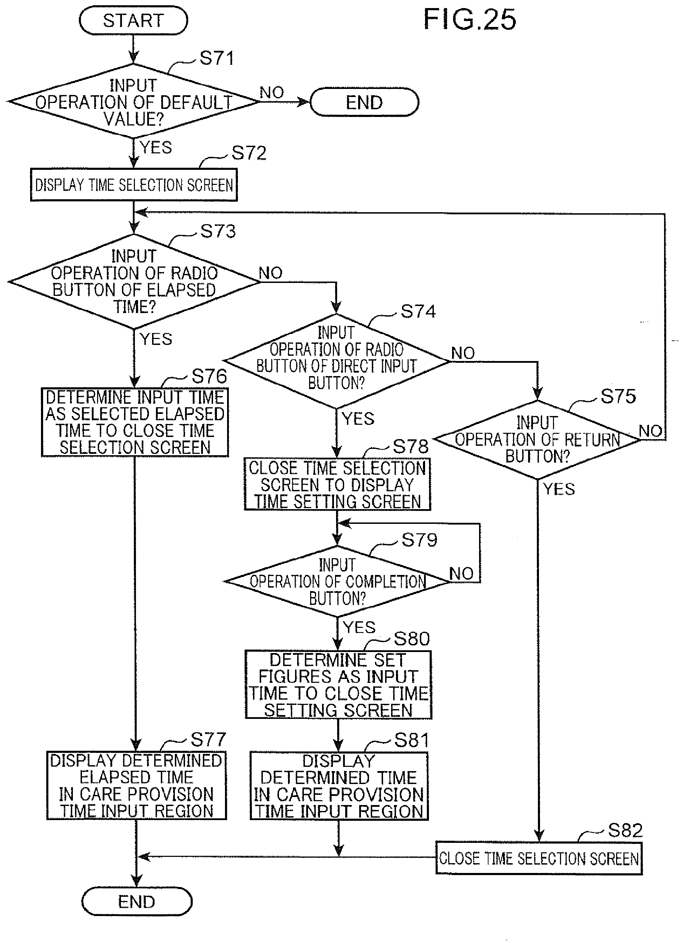

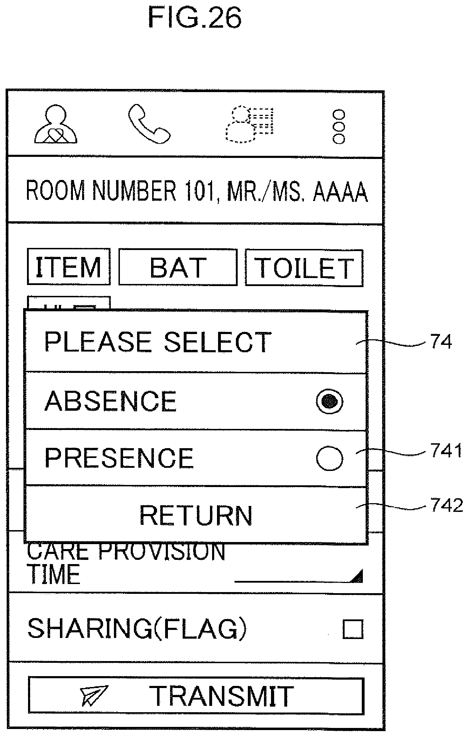

Next, operation of the present embodiment will be described. FIG. 8 is a view showing one example of a standby screen displayed on the mobile terminal device. FIG. 9 is a view showing one example of a monitoring information screen displayed on the mobile terminal device. FIG. 10 is a view showing one example of a nurse call reception screen displayed on the mobile terminal device. FIG. 11 is a flow chart showing operation of selecting a recording form by manual operation in the mobile terminal device. FIG. 12 is a flow chart showing automatic operation of selecting a recording form in the mobile terminal device. FIG. 13 is a flow chart showing automatic operation of selecting a recording form in the management server device. FIG. 14 is a view showing one example of a submenu screen displayed on the mobile terminal device. FIG. 15 is a view showing one example of a target person selection screen displayed on the mobile terminal device. FIG. 16 is a view showing one example of a form selection screen displayed on the mobile terminal device. FIG. 17 is a view showing one example of a meal care recording form screen displayed on the mobile terminal device. FIG. 18 is a view showing one example of a morning care recording form screen displayed on the mobile terminal device. FIG. 19 is a view showing one example of an excretion care recording form screen displayed on the mobile terminal device. FIG. 20 is a view showing one example of a food intake selection screen displayed on the mobile terminal device. FIG. 21 is a view showing one example of a text input screen displayed on the mobile terminal device. FIG. 22 is a view showing one example of a time selection screen displayed on the mobile terminal device. FIG. 23 is a view showing one example of input of care provision time on the meal care recording form screen displayed on the mobile terminal device. FIG. 24 is a view showing one example of a time setting screen displayed on the mobile terminal device. FIG. 25 is a flow chart showing one example of operation of inputting care provision time in the mobile terminal device. FIG. 26 is a view showing one example of a presence/absence input screen displayed on the mobile terminal device.

In the following, first, monitoring operation of a monitored person will be described with reference to FIG. 8 to FIG. 10, secondly, nursing/care recording operation of recording nursing/care in the first mode will be described with reference to FIG. 11, FIG. 14 to FIG. 17, and FIG. 20 to FIG. 25, and thirdly, nursing/care recording operation of recording nursing/care in the second mode will be described with reference to FIG. 12, FIG. 13, FIG. 17 to FIG. 19, and FIG. 26.

In thus configured monitored person monitoring system MS, upon application of power to each device SU, SV, SP, TA, each required portion is initialized to start operation thereof. In the management server device SV, execution of a control processing program thereof functionally configures the SV control portion 221, the SV monitoring processing portion 222, the form distribution processing portion 223, and the SV nursing/care record processing portion 224 in the SV control processing unit 22. In the mobile terminal device TA, execution of a control processing program thereof functionally configures the TA control portion 321, the TA monitoring processing portion 322, the calling processing portion 323, the TA streaming processing portion 324, and the TA nursing/care record processing portion 325 in the TA control processing unit 32.

### (Monitoring Operation)

In the monitoring operation, the sensor device SU operates in the following manner in each frame or every several frames to detect predetermined movement of a monitored person Ob, and to determine reception/non-reception of a nurse call. First, the sensor device SU acquires an image (image data) of one frame as a target image from the image sensor, detects predetermined action of the monitored person Ob on the basis of the acquired target image, and upon detection of the predetermined action, for notifying a predetermined terminal device SP, TA of the detection result, transmits, to the management server device SV, a first event notification communication signal related to detection of the predetermined action and containing the predetermined action as the event information. During such operation, the sensor device SU determines whether a nurse call is accepted or not, and upon acceptance of a nurse call, the sensor device SU transmits, to the management server device SV, a first event notification communication signal related to reception of the nurse call and containing the accepted nurse call as the event information for notifying the predetermined terminal device SP, TA of reception of the nurse call.

Upon receiving the first event notification communication signal from the sensor device SU via the network NW, the management server device SV stores (records), by means of the SV monitoring processing portion 222, each piece of information such as the sensor ID, the event information, and the like contained in the first event notification communication signal as monitoring information of the monitored person Ob monitored by the sensor device SU having the sensor ID. And, the management server device SV specifies, by the SV monitoring processing portion 222, a terminal device SP, TA as a notification destination corresponding to a notification source sensor device SU in the received first event notification communication signal from the notification destination correspondence relationship and transmits a second event notification communication signal to the notification destination terminal device SP, TA.

Upon receiving the second event notification communication signal from the management server device SV via the network NW, the fixed terminal device SP and the mobile terminal device TA store (record), by means of the TA monitoring processing portion 322, each piece of information such as the sensor ID, the event information, and the like contained in the second event notification communication signal as monitoring information of a monitored person Ob monitored by the sensor device SU having the sensor ID, and displays the monitoring information.

More specifically, in the description of operation of the mobile terminal device TA as an exemplification, when power is applied to start operation as described above, the mobile terminal device TA accepts log-in operation by a monitor (user) such as a nurse, a care worker, or the like, for example, so that a standby screen for waiting for a communication signal directed to own device is displayed on the TA display unit 36 by the TA monitoring processing portion 322. The standby screen 51 includes, for example, a menu bar region 511 which displays a menu bar, a standby main region 512 which displays a message (e.g. "no notification") indicative of standby and an icon, a time region 513 which displays current time, a year, month, day, day of the week region 514 which displays year, month, day, and a day of the week of today, and a user name region 515 which displays a user name now logging in the mobile terminal device TA as shown in FIG. 8. When accepting log-in operation, by means of the TA monitoring processing portion 322, the mobile terminal device TA transmits, to the management server device SV, a communication signal (log-in notification communication signal) containing a terminal ID of own device and a name of a monitor (user) having logged in the own device. Upon receiving the log-in notification communication signal, the management server device SV stores, by means of the SV monitoring processing portion 222, the terminal ID and the name of the monitor (monitor information) contained in the received log-in notification communication signal in the SV storage unit 23 so as to be correlated with each other.

During standby for a communication signal directed to own device, the mobile terminal device TA repeats to determine, by the TA control portion 321 of the TA control processing unit 32, whether a communication signal is received by the TA communication IF unit 31 or not until receiving a communication signal, and upon reception of the communication signal, discriminates the type of the received communication signal. As a result of the discrimination, in a case where the received communication signal is not a second event notification communication signal, the mobile terminal device TA conducts appropriate processing according to the received communication signal to end the processing, and by contrast, in a case where the received communication signal is a second event notification communication signal (second event notification), the mobile terminal device TA stores (records), in the TA monitoring information storage portion 331, monitoring information related to monitoring of the monitored person Ob contained in the received second event notification communication signal by means of the TA monitoring processing portion 322 of the TA control processing unit 32, and displays a screen according to each piece of information contained in the received second event notification communication signal on the TA display unit 36.

In more detail, in a case where the event information contained in the received second event notification communication signal is the predetermined action, the TA monitoring processing portion 322 displays the monitoring information screen 52 as shown, for example, in FIG. 9, on the TA display unit 36, and by contrast, in a case where the event information contained in the received second event notification communication signal is the nurse call, displays the nurse call reception screen 53 as shown, for example, in FIG. 10, on the TA display unit 36.

The monitoring information screen 52 is a screen for displaying the monitoring information related to monitoring of a monitored person Ob. The monitoring information screen 52 includes, for example, as shown in FIG. 9, the menu bar region 511, a monitored person name region 521 which displays a disposition place of a sensor device SU having a sensor ID contained in the received second event notification communication signal and a name of a monitored person Ob monitored by the sensor device SU having the sensor ID, a detection information display region 522 which displays elapsed time from reception time (or detection time of the predetermined action) of the received second event notification communication signal, and the event information (a detection result of the predetermined action) contained in the received second event notification communication signal, an image region 523 in which an image (i.e. a target image captured by the sensor device SU having the sensor ID) (still picture here) contained in the received second event notification communication signal is displayed, a "handle" button 524, a "speak" button 525, and a "watch live" button 526.

For displaying a disposition place of a sensor device SU and a name of a monitored person Ob in the monitored person name region 521, a disposition place of the sensor device SU and a name of the monitored person Ob are searched from the TA sensor information storage portion 332 and displayed with the sensor ID contained in the received second event notification communication signal as a search key. While in the detection information display region 522, the detection result (in the present embodiment, each name of wake-up, get-up, fall, and tumble) contained in the received second event notification communication signal may be displayed as it is, an icon symbolically indicating the detection result is displayed in the present embodiment. For displaying the icon, each action and an icon symbolically indicating the action are stored so as to be correlated with each other in the TA storage unit 33 in advance. In the example shown in FIG. 9, a wake-up icon symbolically indicating wake-up is displayed in the detection information display region 522. On the monitoring information screen 52, the "handle" button 524 is a button for inputting, to the mobile terminal device TA, provision intention information indicating that a user of the mobile terminal device TA intends to handle (deal with, cope with) the detection result displayed on the monitoring information screen 52 by providing predetermined nursing/care such as life-saving, nursing, care and assistance. The "speak" button 525 is a button for requesting voice calling and for inputting an instruction to communicably connect the sensor device SU with the sensor ID and the mobile terminal device TA via the network NW. The "watch live" button 526 is a button for requesting live moving picture and for inputting an instruction to display moving picture captured by the sensor device SU with the sensor ID.

The nurse call reception screen 53 is a screen for displaying reception of a nurse call. The nurse call reception screen 53 includes, for example, as shown in FIG. 10, the menu bar region 511, the monitored person name region 521, the detection information display region 522, a nurse call reception notification display region 531 which displays a message (e.g. "nurse call") indicating that a nurse call is accepted, the "handle" button 524, and the "speak" button 525. On the nurse call reception screen 53, in the detection information display region 522, only elapsed time from reception time of the received second event notification communication signal (or reception time when the nurse call is accepted) is displayed. The nurse call reception screen 53 may further include the "watch live" button 526.

And, during displaying of such monitoring information screen 52 or nurse call reception screen 53, the mobile terminal device TA repeats the operation until accepting input operation by the TA control processing unit 32, and determines whether input operation is accepted or not by the touch panel formed with the TA input unit 35 and the TA display unit 36, and when accepting input operation, the mobile terminal device TA executes appropriate processing according to contents of the input operation by the TA control processing unit 32 to end the present processing.

For example, when accepting input operation of the "handle" button 524 from the TA input unit 35 forming the touch panel by the TA control processing unit 32 (i.e. accepting the intention to handle), the mobile terminal device TA first stores monitoring information of the monitored person Ob currently displayed on the TA display unit 36 in the TA monitoring information storage portion 331, the monitoring information being applied an effect that "to handle" has been accepted. More specifically, the TA control processing unit 32 registers, in the TA monitoring information table MT-TA stored in the TA monitoring information storage portion 331, the handling flag "1" indicative of reception of handling in a handling field 3316 of a record in which monitoring information of the monitored person Ob currently displayed on the TA display unit 36 is registered (here, a record in which monitoring information contained in the received second event notification communication signal is registered). And, the TA control processing unit 32 transmits a communication signal (handling reception notification communication signal) to the management server device SV, the communication signal containing a sensor ID corresponding to the monitoring information of the monitored person Ob displayed on the TA display unit 36 and information indicating that "to handle" is accepted (handling reception information). The management server device SV having received the handling reception notification communication signal transmits, to the terminal device SP, TA by broadcast communication by the SV control processing unit 22, a communication signal (handling reception publicizing communication signal) containing a sensor ID and the handling reception information contained in the received handling reception notification communication signal. As a result, regarding the sensor ID corresponding to monitoring information of the monitored person Ob displayed on the TA display unit 36, the effect that "to handle" has been accepted is synchronized between the terminal devices SP and TA.

For example, in the mobile terminal device TA, upon accepting input operation of the "speak" button 525 by the TA control processing unit 32, the calling processing portion 323 transmits a communication signal (calling request communication signal) containing information that voice calling is to be requested to the sensor device SU which monitors a monitored person Ob displayed on the TA display unit 36, so that the sensor device SU is responsively connected to enable voice calling via the network NW. As a result, voice calling is allowed between the mobile terminal device TA and the sensor device SU. In the mobile terminal device TA, upon accepting input operation of an "end" button not shown by the TA control processing unit 32, the button being for inputting an instruction to end voice calling, the calling processing portion 323 transmits, to the sensor device SU which monitors the monitored person Ob displayed on the TA display unit 36, a communication signal (calling end communication signal) containing information that end of voice calling is to be requested. As a result, voice calling between the mobile terminal device TA and the sensor device SU ends.

For example, in the mobile terminal device TA, upon accepting input operation of the "watch live" button 526 by the TA control processing unit 32, the TA streaming processing portion 324 transmits a communication signal (moving picture distribution request communication signal) containing information that distribution of live moving picture is to be requested to the sensor device SU which currently monitors a monitored person Ob displayed on the TA display unit 36, so that the sensor device SU is responsively connected to enable down-load of moving picture via the network NW, thereby receiving distribution of live moving picture from the sensor device SU and displaying the distributed moving picture on the TA display unit 36 by streaming reproduction. On the monitoring information screen 52 which displays the live moving picture, moving picture is displayed in the image region 523, and then, a "live end" button not shown is displayed in place of the "watch live" button 526. As a result, live moving picture is displayed in the mobile terminal device TA. The "live end" button not shown is a button for requesting end of moving picture, the button being for inputting an instruction to end (stop) distribution of moving picture captured by the sensor device SU with the sensor ID to end (stop) display. Upon accepting input operation of the "live end" button by the TA control processing unit 32, the mobile terminal device TA transmits a communication signal (moving picture distribution end communication signal) containing information that end of moving picture distribution is to be requested to the sensor device SU which currently monitors the monitored person Ob currently displayed on the TA display unit 36 by a TA streaming processing portion 324, and displays still picture on the TA display unit 36. As a result, the mobile terminal device TA ends displaying of live moving picture.

By such operation, the monitored person monitoring system MS roughly detects predetermined action of each monitored person Ob and accepts a nurse call to monitor each monitored person Ob by each sensor device SU, the management server device SV, the fixed terminal device SP, and the mobile terminal device TA.

### (Nursing/Care Recording Operation in First Mode)

In the nursing/care recording operation in the first mode, upon accepting input operation of a submenu button 5111 in the menu bar region 511 on the standby screen 51, the monitoring information screen 52, the nurse call reception screen 53, or the like from the TA input unit 35 forming the touch panel (S11) in FIG. 11, the mobile terminal device TA displays a submenu screen 61 shown, for example, in FIG. 14, on the TA display unit 36 by the TA nursing/care record processing portion 325 (S12).

The submenu button 5111 is a button for inputting an instruction to display the submenu screen 61 to the mobile terminal device TA. The submenu screen 61 is a screen for displaying selectable submenus to select a submenu. The submenu screen 61 includes an "information" button 611, a "staff message board" button 612, a "care provision input" button 613, and a "log-out" button 614 as submenus as shown, for example, in FIG. 14. The "information" button 611 is a button for inputting, to the mobile terminal device TA, an instruction to display predetermined information such as a log-in name (care worker name), a terminal ID, and the like. The "staff message board" button 612 is a button for inputting, to the mobile terminal device TA, an instruction to display a message recorded in the management server device SV by a monitor (user) from the terminal device SP, TA. The "care provision input" button 613 is a button for inputting, to the mobile terminal device TA, an instruction to record a nursing/care record on the management server device SV by a monitor from the terminal device SP, TA. The "log-out" button 614 is a button for inputting, to the mobile terminal device TA, an instruction to log out.

Next, the mobile terminal device TA discriminates whether input operation is accepted from the TA input unit 35 forming the touch panel by means of the TA control processing unit 32 or not (S13). As a result of the discrimination, in a case where no input operation is accepted (No), the mobile terminal device TA returns the processing to the processing of S13, and by contrast, as a result of the discrimination, in a case where input operation is accepted, the mobile terminal device TA executes next processing of S14.

In the processing of S14, by means of the TA control processing unit 32, the mobile terminal device TA discriminates the type of the input operation accepted at the TA input unit 35 forming the touch panel. As a result of the discrimination, in a case where the input operation accepted in the processing of S13 is the "care provision input" button 613 (care provision input), the mobile terminal device TA executes next processing of S15, and in a case where the input operation accepted in the processing of S13 is not the "care provision input" button 613 (other), executes processing of S22 of conducting appropriate processing according to input operation accepted in the processing of S13, then displays the screen (the standby screen 51, the monitoring information screen 52, the nurse call reception screen 53, or the like) on the TA display unit 36, the screen displaying the submenu button 5111 accepted in the processing of S 11, and ends the present processing.

In the processing of S15, the mobile terminal device TA displays, on the TA display unit 36, a target person selection screen 62 shown, for example, in FIG. 15, by the TA nursing/care record processing portion 325. The target person selection screen 62 is a screen for displaying selectable target persons to select a target person of a nursing/care record subjected to nursing/care. The target person selection screen 62 includes, for example, as shown in FIG. 15, the menu bar region 511, a submenu name display region 621 for displaying a submenu name selected on the submenu screen 61, and a target person display region 622 (622-1 to 622-5) displaying a list of one or a plurality of selectable target persons. In this example, in the submenu name display region 621, submenu name; "care provision input" of a submenu corresponding to the "care provision input" button 613 accepted in the processing of S13 is displayed. For displaying selectable target persons in a list in the target person display region 622, a monitored person name and a disposition place registered in a monitored person name field 3323 and a disposition place field 3322 in the TA sensor information table ST-TA stored in the TA sensor information storage portion 332 are extracted from each record, and the disposition place and the monitored person name are sorted in a set (pair) according to a predetermined criterion in order from the top to down in a front view of the TA display unit 36 and displayed in a list in the target person display region 622. Any sorting criterion may be employed and, for example, such data may be displayed in the order of the Japanese syllabary (alphabetical order) of monitored person names, in the order of room numbers at a disposition place, or the like. In the example shown in FIG. 15, the sets of a disposition place and a monitored person name are sorted in the order of room numbers at a disposition place. In a case where the target person display region 622 is larger than a display region of the TA display unit 36, and each set of a disposition place and a monitored person name cannot be all displayed in the display region of the TA display unit 36, an area of the target person display region 622 displayed in the display region of the TA display unit 36 is changed by, for example, flick (flick input), so that a set of a disposition place and a monitored person name not displayed in the display region of the TA display unit 36 can be displayed. Then, the individual regions 622-1 to 622-5 in which each set of a disposition place and a monitored person name is displayed are also buttons (target person selection button) for inputting, to the mobile terminal device TA, a target selected by a monitor (user of the mobile terminal device TA).

Next, during displaying of the target person selection screen 62, when accepting, from the TA input unit 35 forming the touch panel, input operation of a region (target person selection button) 622 in which a set of a disposition place and a monitored person name is displayed to accept selection of a target person (S16), the mobile terminal device TA stores, by the TA nursing/care record processing portion 325, the selected target person (monitored person name) in the TA storage unit 33 as a target person of a nursing/care record and displays a form selection screen 63 shown in, for example, FIG. 16, on the TA display unit 36 (S17). The form selection screen 63 is a screen for displaying selectable recording forms prepared in advance according to the type of nursing/care and selecting a recording form. The target person selection screen 62 includes the menu bar region 511, a target person name display region 631 which displays a target person name selected on the target person selection screen 62, and a form display region 632 (632-1 to 632-4) which displays one or a plurality of selectable recording forms in a list of names as shown, for example, in FIG. 16. In the example shown in FIG. 16, input operation of the region (target person selection button) 622-1 for displaying "room number 101 AAAA" is conducted in the processing of S16, so that "room number 101 Mr./Ms. AAAA" is displayed in the target person name display region 631. The target person name display region 631 is also a button for inputting an instruction to display the target person selection screen 62 to the mobile terminal device TA. Accordingly, input operation of the target person name display region 631 enables change of a target person on the target person selection screen 62. In a case where the form display region 632 is larger than the display region of the TA display unit 36, so that all the recording form names cannot be displayed in the display region of the TA display unit 36, an area of the form display region 632 which is displayed in the display region of the TA display unit 36 is changed, for example, by flick (flick input) so that a recording form name which is not displayed in the display region of the TA display unit 36 can be displayed. And, the individual regions 632-1 to 632-4 which display the recording form names are also buttons (form selection button) for inputting a recording form selected by a monitor to the mobile terminal device TA. In the example shown in FIG. 16, the form display region 632 has the "meal" region 632-1 for displaying "meal" as a recording form name for meal care, the "hydration" region 632-2 for displaying "hydration" as a recording form name for hydration care, the "excretion" region 632-3 for displaying "excretion" as a recording form name for excretion care, and the "vital" region 632-4 for displaying "vital" as a recording form name for vital check. And, the "meal" region 632-1 is also a button for inputting selection of the recording form for meal care to the mobile terminal device TA, the "hydration" region 632-2 is also a button for inputting selection of the recording form for hydration care to the mobile terminal device TA, the "excretion" region 632-3 is also a button for inputting selection of the recording form for excretion care to the mobile terminal device TA, and the "vital" region 632-4 is also a button for inputting selection of the recording form for vital check to the mobile terminal device TA. Further, in the example shown in FIG. 16, contents of nursing/care records are displayed in the individual regions 632-1 to 632-4 which display the recording form names. For example, each content of a nursing/care record of today, of previous day, or a previous nursing/care record is displayed. For displaying each content of a nursing/care record in the region 632, the TA nursing/care record processing portion 325 transmits, to the management server device SV, a communication signal (a nursing/care record request communication signal) containing own device terminal ID, a target person name (monitored person name) selected and input in the processing of S16, and an instruction to request each content of a nursing/care record of today, of previous day, or a previous nursing/care record. Upon receiving the nursing/care record request communication signal, the management server device SV extracts, from the nursing/care record information storage portion 235, each content of a nursing/care record of today, of previous day, or a previous nursing/care record corresponding to the target person name (monitored person name) contained in the received nursing/care record request communication signal, and returns, to the mobile terminal device TA as a transmission source of the received nursing/care record request communication signal, the communication signal (nursing/care record return communication signal) containing the extracted each content of a nursing/care record of today, of previous day, or a previous nursing/care record, and the terminal ID and the target person name (monitored person name) contained in the received nursing/care record request communication signal. The TA nursing/care record processing portion 325 receives the nursing/care record return communication signal from the management server device SV and extracts the contained data to display each contents of the nursing/care record in the region 632.

Next, upon accepting input operation of the region (form selection button) 632 in which a recording form name is displayed from the TA input unit 35 forming the touch panel to accept selection of a recording form name (S18) during displaying of the form selection screen 63, the mobile terminal device TA transmits, by means of the TA nursing/care record processing portion 325, a communication signal (third form request communication signal) containing own device terminal ID, the selected recording form name and, and an instruction to request a recording form of the relevant recording form name to the management server device SV via the TA communication IF unit 31 (one example of an input side communication unit), thereby requesting the management server device SV for the recording form the selection of which has been accepted (S19).

Upon receiving the third form request communication signal, the SV control portion 221 (one example of a response control portion) of the management server device SV extracts an electronic file in a recording form corresponding to the recording form name contained in the received third form request communication signal from the form information storage portion 234 to return a communication signal (third form return communication signal) containing the extracted electronic file, and the terminal ID and the recording form name contained in the received third form request communication signal to the mobile terminal device TA as a transmission source of the received third form request communication signal via the SV communication IF unit 21 (one example of a server side communication unit).

Next, upon receiving the third form return communication signal from the management server device SV during displaying of the form selection screen 63, the mobile terminal device TA extracts the electronic file in the recording form contained in the received third form return communication signal and displays a recording form screen 64 on the TA display unit 36 using the extracted electronic file in the recording form (S20, one example of a display step).

For example, when input operation of the region (form selection button) 632-1 for displaying "meal "is conducted in the processing of S18, the mobile terminal device TA (one example of a care item input device) transmits a third form request communication signal for requesting a recording form for meal care to the management server device SV by means of the TA nursing/care record processing portion 325, and as a response, receives a third return form communication signal containing an electronic file in a recording form for meal care to display, for example, a meal care recording form screen 64a as shown in FIG. 17 on the TA display unit 36 (one example of a display unit).

The meal care recording form screen 64a (one example of a display screen) is a screen for displaying a recording form for meal care, a screen for inputting contents of conducted meal care as a nursing/care record. The meal care recording form screen 64a includes, as shown, for example, in FIG. 17, the menu bar region 511, the target person name display region 631 for displaying a target person name, a nursing/care record input region 641a which displays an input item to be input as nursing/care contents to input nursing/care contents according to the input item as a nursing/care record, a sharing check box (□) 642 for inputting setting to allow or not to allow reference of the nursing/care record from other terminal device SP, TA, and a "transmit" button 643.

In the meal care recording form in the present embodiment, the input items are, for example, an intake of staple food, an intake of a side dish, memos, and care provision time, and for inputting these items, the nursing/care record input region 641a has a staple food amount input region 641a-1 for inputting a staple food amount, a side dish amount input region 641a-2 for inputting a side dish amount, a free remarks input region 641a-3 for inputting a memo, and a care provision time input region 641a-4 for inputting the time when nursing/care was provided. In the sharing check box 642, when input operation of a check mark is accepted, "√" is displayed in the sharing check box 642 to input an instruction to set such that the contents input to the recording form screen 64 (in the example shown in FIG. 17, the relevant meal care recording form screen 64a) can be referred to by other terminal device SP, TA. By contrast, when input operation of a check mark is not accepted, the sharing check box 642 is displayed in blank to input an instruction to set such that the contents input to the recording form screen 64 (in the example shown in FIG. 17, the relevant meal care recording form screen 64a) cannot be referred to by other terminal device SP, TA. The "transmit" button 643 is a button for inputting an instruction to transmit the contents (in the example shown in FIG. 17, meal care contents) input to the recording form screen 64 (in the example shown in FIG. 17, the relevant meal care recording form screen 64a) to the management server device SV.

As shown in FIG. 17, in the care provision time input region 641a-4 on the meal care recording form screen 64a, default value of "present" is displayed. In a case where input operation of the care provision time input region 641a-4 is not conducted by a monitor (user) in the subsequent S21, the TA control portion 321 applies the default value of "present" as care provision time.

Returning to FIG. 11, next, the mobile terminal device TA executes recording form processing of transmitting nursing/care contents input by using a recording form displayed on the TA display unit 36 as a nursing/care record to the management server device SV by means of the TA nursing/care record processing portion 325 (S21, one example of an accepting step, one example of display controlling step), and thereafter displays, on the TA display unit 36, a screen (the standby screen 51, the monitoring information screen 52, the nurse call reception screen 53, or the like) on which the submenu button 5111 accepted in the processing of S11 is displayed to end the present processing.

More specifically, the monitor (user) first refers to a recording form displayed on the TA display unit 36 to input nursing/care contents according to an input item of the recording form.

In the above example, the monitor (user) refers to the meal care recording form screen 64a displayed on the TA display unit 36 to input meal care contents in each of the regions 641a-1 to 641a-4 in the nursing/care record input region 641a on the meal care recording form screen 64a by using the TA input unit 35, and conducts input operation of the sharing check box (□) 642 as required.

In more detail, in the present embodiment, upon input operation (e.g. tapping) of each of the regions 641a-1 to 641a-4 in the nursing/care record input region 641a on the meal care recording form screen 64a shown in FIG. 17, a sub-window for inputting data according to an attribute (e.g. text data, numerical value data) of data to be input to the relevant regions 641a-1 to 641a-4 is opened to be overlapped with the relevant meal care recording form screen 64a.

For example, when input operation of the staple food amount input region 641a-1 or the side dish amount input region 641a-2 is conducted, for example, a food intake selection screen 71 shown in FIG. 20 is displayed on the TA display unit 36 so as to be overlapped with the relevant meal care recording form screen 64a. The food intake selection screen 71 is a screen for selectively inputting a ratio of meal taken by a monitored person Ob relative to a total meal intake (a total amount of staple food and a total amount of side dish) to the relevant mobile terminal device TA. The food intake selection screen 71 includes, for example, as shown in FIG. 20, an intake ratio selection region 711 in which a plurality of different intake ratios that can be selectively input is displayed, and a "return" button 712. Each region in the intake ratio selection region 711 where each intake ratio is displayed is provided with a plurality of radio buttons (o) for designating and inputting one intake ratio from among the plurality of intake ratios. In the example shown in FIG. 20, a radio button of a ratio of 0 is checked, so that the ratio of 0 is designated and input to the mobile terminal device TA. The "return" button 712 is a button for inputting an instruction to close the sub-window (the food intake selection screen 71 in the example shown in FIG. 20).

For example, when input operation of the free remarks input region 641a-3 is conducted, a text input screen 72, for example, as shown in FIG. 21, is displayed on the TA display unit 36 so as to be overlapped with the relevant meal care recording form screen 64a. The text input screen 72 is a screen for inputting letters to the relevant mobile terminal device TA. The text input screen 72 includes, for example, as shown in FIG. 21, a letter input keyboard screen 721 provided with a plurality of different letter buttons that can be selectively input, and the like.

For example, when input operation of the default value of the care provision time input region 641a-4 is conducted using the TA input unit 35 forming the touch panel (one example of an operation input unit), the TA control portion 321 (one example of a display control portion) displays, for example, a time selection screen 75 (one example of an input screen) as shown in FIG. 22 on the TA display unit 36 so as to be overlapped with the relevant meal care recording form screen 64a. The time selection screen 75 is a screen for selecting time and inputting the same to the relevant mobile terminal device TA. The time selection screen 75 is provided with, for example, as shown in FIG. 22, an elapsed time selection region 751 in which a plurality of different and selectable elapsed times is displayed, a direct input button 752 for selecting direct input of a numerical value, and a "return" button 753. The "return" button 753 is a button for inputting an instruction to close the sub-window (the time selection screen 75 in the example shown in FIG. 22).

Each elapsed time displayed in the elapsed time selection region 751 indicates an elapsed time dating back from current time. Specifically, "present" represents, for example, that meal care provision time is the current time, and "1 hour ago" represents that meal care provision time is one hour ago the current time. Each region in the elapsed time selection region 751 displaying each elapsed time is provided with a radio button (o) for designating and inputting one elapsed time from among the plurality of elapsed times. For example, as shown in FIG. 22, when a radio button of "15 minutes ago" is checked, input of care provision time is finalized to designate "15 minutes ago" and input the same to the mobile terminal device TA, so that the sub-window (the time selection screen 75 in the example shown in FIG. 22) is closed. As a result, the meal care recording form screen 64a is displayed in which "15 minutes ago" is displayed in the care provision time input region 641a-4 as shown in FIG. 23.

While in FIG. 22, the radio button of "15 minutes ago" is checked, in a state immediately after display of the meal care recording form screen 64a on the TA display unit 36, a radio button of "present" as a default value is checked.

The direct input button 752 is an option which enables direct input of meal care provision time as a numerical value. When input operation (e.g. tapping) of a radio button corresponding to the direct input button 752 is conducted, the time selection screen 75 is closed to display a time setting screen 76 shown in FIG. 24 on the TA display unit 36 so as to be overlapped with the meal care recording form screen 64a.

The time setting screen 76 has a time setting region 761, a minute setting region 762, and a "completion" button 763. When scrolling upward or downward on the time setting region 761 and the minute setting region 762 using, for example, the TA input unit 35 forming the touch panel, figures are individually scrolled upward or downward in the time setting region 761 and the minute setting region 762. The time setting screen 76 shown in FIG. 24 is set to be "11:14".

When in the state shown in FIG. 24, input operation (e.g. tapping) of the "completion" button 763 is conducted using the TA input unit 35 forming the touch panel, input of care provision time is finalized to designate "11:14" and input the same to the mobile terminal device TA, so that the sub-window (the time setting screen 76 in the example shown in FIG. 24) is closed. As a result, similarly to FIG. 23, the meal care recording form screen 64a on which "11:14" is displayed in the care provision time input region 641a-4 is displayed on the TA display unit 36.

The monitor (user) inputs a staple food intake to the staple food amount input region 641a-1 from the TA input unit 35 forming the touch panel by using the food intake selection screen 71, inputs a side dish intake to the side dish amount input region 641a-2 from the TA input unit 35 by using the food intake selection screen 71, inputs a memo as required to the free remarks input region 641a-3 from the TA input unit 35 by using the text input screen 72, inputs the time when nursing/care was provided to the care provision time input region 641a-4 from the TA input unit 35 by using the time selection screen 75 or the time setting screen 76, and conducts input operation of the sharing check box (□) 642 as required.

And, the monitor (user) conducts input operation of the "transmit" button 643.

Upon accepting the input operation of the "transmit" button 643, the mobile terminal device TA transmits, to the management server device SV, a communication signal (nursing/care record communication signal) containing the own device terminal ID, the target person name (monitored person name) selected and input in the processing of S16, and contents of a nursing/care record input by using the recording form screen 64 (in the above example, the staple food intake, the side dish intake, the memos, the care provision time, and sharing allowed/not allowed which are input using the meal care recording form screen 64a) via the TA communication IF unit 31 (one example of an input side communication unit) by means of the TA nursing/care record processing portion 325 (one example of a transmission control portion).

Upon receiving the nursing/care record communication signal via the SV communication IF unit 21 (one example of a server side communication unit), the management server device SV stores the target person name (monitored person name) and the contents of the nursing/care record (in the above example, the staple food intake, the side dish intake, the memos, the care provision time, and sharing allowed/not allowed) contained in the received nursing/care record communication signal in the nursing/care record information storage portion 235 (one example of a server side storage portion) so as to be correlated with each other.

At this time, in a case where "present" is transmitted from the mobile terminal device TA, the SV control portion 221 (one example of a management control portion) of the management server device SV considers reception time of the nursing/care record communication signal as care provision time. In a case where "dating back elapsed time", e.g. "15 minutes ago" is transmitted as care provision time from the mobile terminal device TA, the management server device SV also considers 15 minutes before the reception time of the nursing/care record communication signal as care provision time. In a case where care provision time directly input using the time setting screen 76 (FIG. 24) is transmitted from the mobile terminal device TA, the management server device SV considers the transmitted care provision time as it is as care provision time.

In the nursing/care recording operation in the first mode, each processing is thus executed to input a nursing/care record to the mobile terminal device TA by using a recording form and is stored (recorded) in the management server device SV.

With such operation as described above, the monitored person monitoring system MS (one example of a care item management system) stores and manages a result of nursing/care (one example of care) conducted for a monitored person Ob by means of each sensor device SU, the management server device SV, the fixed terminal device SP, and the mobile terminal device TA.

Here, description will be made of an input procedure of care provision time, the procedure being started in a state where the meal care recording form screen 64a shown in FIG. 17 is displayed on the TA display unit 36 of the mobile terminal device TA, with reference to the flow chart of FIG. 25. Operation in FIG. 25 is executed every fixed time (e.g. 100 msec) in a state where the meal care recording form screen 64a shown in FIG. 17 is displayed on the TA display unit 36.

In FIG. 25, first, the TA control portion 321 determines whether input operation (e.g. tapping) of the default value ("present" in FIG. 17) displayed in the care provision time input region 641a-4 of the meal care recording form screen 64a is conducted by using the TA input unit 35 or not (S71). When input operation of the default value is not conducted (NO in S71), the operation in FIG. 25 ends.

By contrast, when input operation of the default value is conducted (YES in S71), the TA control portion 321 displays the time selection screen 75 shown in FIG. 22 on the TA display unit 36 so as to be overlapped with the meal care recording form screen 64a (S72).

Next, the TA control portion 321 determines whether input operation of the radio button in the elapsed time selection region 751 is conducted on the time selection screen 75 or not (S73). When no input operation of the radio button in the elapsed time selection region 751 is conducted (NO in S73), the TA control portion 321 determines whether input operation of a radio button corresponding to the direct input button 752 is conducted or not (S74). When no input operation of the radio button corresponding to the direct input button 752 is conducted (NO in S74), the TA control portion 321 determines whether input operation of the return button 753 is conducted or not (S75). When no input operation of the return button 753 is conducted (NO in S75), the processing returns to the processing of S73. Thus, in the processing of S73, S74, and S75, user's input operation of the time selection screen 75 is waited for.

When in the processing of S73, input operation of the radio button in the elapsed time selection region 751 is conducted (YES in S73), the TA control portion 321 determines elapsed time corresponding to the input radio button as selected elapsed time to close the time selection screen 75 (S76). Subsequently, the TA control portion 321 displays the determined elapsed time ("15 minutes ago" in the examples in FIG. 22 and FIG. 23) in place of the default value in the care provision time input region 641a-4 on the meal care recording form screen 64a (S77). With the processing of S77, the operation of FIG. 25 ends.

When in the processing of S74, input operation of the radio button corresponding to the direct input button 752 is conducted (YES in S74), the TA control portion 321 closes the time selection screen 75 to display the time setting screen 76 shown in FIG. 24 on the TA display unit 36 so as to be overlapped with the meal care recording form screen 64a (S78).

Next, the TA control portion 321 determines whether input operation of the "completion" button 763 is conducted on the time setting screen 76 or not (S79). When the input operation of the "completion" button 763 is not conducted (NO in S79), input operation of the "completion" button 763 is waited for in the processing of S79 until conducted. Meanwhile, the monitor (user) scrolls the time setting region 761 and the minute setting region 762 to set a desired figure. When input operation of the "completion" button 763 is conducted (YES in S79), the TA control portion 321 determines figures set at that time in the time setting region 761 and the minute setting region 762 as input time to close the time setting screen 76 (S80). Subsequently, the TA control portion 321 displays the input time (11:14 in the example of FIG. 24) in place of the default value in the care provision time input region 641a-4 on the meal care recording form screen 64a (S81). The processing of S81 completes the operation of FIG. 25.

When input operation of the return button 753 is conducted in the processing of S75 (YES in S75), the TA control portion 321 closes the time selection screen 75 (S82). The processing of S82 completes the operation of FIG. 25.

Thus, in the present embodiment, a default value is displayed in the care provision time input region 641a-4 on the meal care recording form screen 64a, and in a case where the default value is not changed, the default value is applied. Accordingly, in a case, for example, where the monitor (user) inputs each item of the meal care recording form screen 64a immediately after meal care is provided, "present" as a default value for care provision time can be used. Therefore, input of the care provision time input region 641a-4 can be omitted. As a result, monitor's load (user's load) of input work can be mitigated.

Also in the present embodiment, when input operation of a default value is conducted, the time selection screen 75 including the elapsed time selection region 751 is displayed. This allows a monitor (user) to select elapsed time dating back from current time as care provision time.

Regarding timing of past action, in a case of going back in memory to estimate time, it is in general easier for human brain to memorize "how many minutes ago action was taken from now" rather than "about what time action was taken". The order of action is also easy to memorize. Therefore, in a case of inputting memorized time related to a plurality of works, it is easier to memorize "first Mr./Ms. AAAA, oo minutes ago, then, next Mr./Ms. BBBB, □□ minutes ago because Mr./Ms. AAAA took about ΔΔ minutes, and then Mr./Ms. CCCC ..." rather than "Mr./Ms. AAAA, at around oo:oo, next Mr./Ms. BBBB, at around □□:xx, next Mr./Ms. CCCC, ...", and time to be specified is also more accurate. Accordingly, the present embodiment enables care provision time to be input more easily and more accurately.

Further, the present embodiment is configured, as an input mode of elapsed time, to select one from a plurality of options of "○ minute ago" included in the elapsed time selection region 751 and to input the same. Therefore, the present embodiment enables a large time gap to be prevented which is generated due to erroneous input occurring at direct input of figures using, for example, ten keys. Specifically, such errors can be prevented as inputting "13" while thinking about "3" in mind, or erroneously inputting only one of figures simply by mistake while intending to input right figures.

### (Nursing/Care Recording Operation in Second Mode)

First, description will be made of the nursing/care recording operation in the mobile terminal device TA in the second mode. In FIG. 12, upon accepting predetermined input operation for requesting a recording form from the TA input unit 35 forming the touch panel (S31), the mobile terminal device TA acquires a status parameter by the TA nursing/care record processing portion 325 (S32). The predetermined input operation for requesting a recording form is the remaining input operation excluding input operation of the submenu button 5111, and in the present embodiment, includes, for example, tapping during displaying of the standby screen 51, tapping during displaying of the monitoring information screen 52, and tapping during displaying of the nurse call reception screen 53. In the present embodiment, as described above, since monitor information in a status parameter is transmitted to the management server device SV from the mobile terminal device TA at the time of log-in, and time for requesting the recording form in the status parameter can be determined (acquired) by the management server device SV, a status parameter acquired in the processing of S32 is position information of the relevant mobile terminal device TA. The position information of the relevant mobile terminal device TA is obtained from each reception intensity of each communication signal received from each of the plurality of access points AP based on the principle of so-called trigonometrical survey because, for example, a reception intensity of a communication signal is proportional to a distance. More specifically, the mobile terminal device TA conducts broadcast communication of communication signals for requiring a communication signal (ACK) of reception acknowledgement by means of the TA nursing/care record processing portion 325, and the access point AP having received the communication signal returns ACK containing own device identifier (ID), so that the mobile terminal device TA obtains each reception intensity of each ACK from the plurality of access points by means of the TA nursing/care record processing portion 325. Additionally, the mobile terminal device TA may include, for example, a gyro sensor to obtain a present position as the position information by obtaining a movement locus from an initially set position on the basis of output of the gyro sensor. The mobile terminal device TA may also include, for example, a GPS (Global Positioning System) device to obtain a present position as the position information by using the GPS device.

Next, the mobile terminal device TA transmits a first form request communication signal to the management server device SV by means of the TA nursing/care record processing portion 325, the first form request communication signal containing own device terminal ID, the status parameter acquired in the processing of S32 (position information (e.g. a plurality of pairs of an ID of an access point AP and a reception intensity of ACK received from the access point AP) in the present embodiment), and an instruction requesting a recording form (S33).

By thus operating as will be described later, the management server device SV having received the first form request communication signal transmits a first form return communication signal containing an electronic file in a recording form selected on the basis of the status parameter contained in the first form request communication signal to the mobile terminal device TA as a transmission source of the received first form request communication signal.

Next, upon receiving the first form return communication signal from the management server device SV as a response to the first form request communication signal transmitted in the processing of S33, the mobile terminal device TA extracts the electronic file in the recording form contained in the received first form return communication signal by means of the TA nursing/care record processing portion 325, and displays the recording form screen 64 on the TA display unit 36 using the extracted electronic file in the recording form (S34). In the target person name display region 631 of the recording form screen 64, a target person name is displayed according to a predetermined rule set in advance. According to the predetermined rule, a target person may be, for example, a top monitored person of monitored person names sorted in the order of the Japanese syllabary (alphabetical order), may be, for example, a monitored person corresponding to a top disposition place among disposition places sorted in the order of room numbers, and may be, for example, when the position of the mobile terminal device TA is a disposition place, a monitored person corresponding to the disposition place. Thus, a recording form (first candidate recording form) is displayed on the TA display unit 36 on the basis of a status parameter only by requesting a recording form.

Next, the mobile terminal device TA determines whether during displaying of such a first candidate recording form, predetermined input operation for requesting another recording form is accepted or not (S35). In a case where the first candidate recording form is a desired recording form, the user records nursing/care by using the first candidate recording form. Specifically, in a case where the TA nursing/care record processing portion 325 accepts input operation for the recording form displayed on the TA display unit 36, such as the above-described opening of a sub-window, the mobile terminal device TA determines that the predetermined input operation for requesting another recording form is not accepted (No) and executes processing of S40. By contrast, in a case where the first candidate recording form is not a desired recording form, the user conducts predetermined input operation for requesting another recording form (a recording form (second candidate recording form) different from the first candidate recording form). In the present embodiment, the predetermined input operation for requesting another recording form is, for example, double tapping during displaying of the recording form screen 64, or sliding during displaying of the recording form screen 64. In other words, the mobile terminal device TA executes the processing of S36 when the predetermined input operation for requesting another recording form is accepted by the TA nursing/care record processing portion 325 (Yes).

In the processing of S36, the mobile terminal device TA transmits, to the management server device SV, a second form request communication signal containing own device terminal ID, and a second instruction to request another recording form by means of the TA nursing/care record processing portion 325.

By operating in a manner as will be described later, the management server device SV, having received the second form request communication signal, selects a second recording form (second candidate recording form) different from the recording form (first candidate recording form) selected for the first form return communication signal on the basis of the status parameter contained in the first form request communication signal, and transmits a second form return communication signal containing an electronic file in the selected second recording form to the mobile terminal device TA as a transmission source of the received second form request communication signal.

Next, upon receiving the second form return communication signal from the management server device SV as a response to the second form request communication signal transmitted in the processing of S36, the mobile terminal device TA extracts the electronic file in the recording form contained in the received second form return communication signal by the TA nursing/care record processing portion 325 to display the recording form screen on the TA display unit 36 using the extracted electronic file in the recording form (S37). In the target person name display region 631 of the recording form screen 64, a target person name is displayed according to a predetermined rule set in advance similarly to the above. Thus, in a case where a first candidate recording form is not a desired recording form, just by requesting a recording form, another recording form (second candidate recording form) is further displayed on the TA display unit 36 on the basis of a status parameter.

Next, the mobile terminal device TA determines whether input operation of the submenu button 5111 is accepted during displaying of such a second candidate recording form or not (S38). In a case where the second candidate recording form is a desired recording form, the user records nursing/care by using the second candidate recording form. Specifically, in a case where the TA nursing/care record processing portion 325 accepts input operation for the recording form displayed on the TA display unit 36, such as the above-described opening of a sub-window, the mobile terminal device TA determines that input operation of the submenu button 5111 is not accepted (No) and executes the processing of S40. By contrast, in a case where the second candidate recording form is not a desired recording form, the user conducts input operation of the submenu button 5111 for requesting a desired recording form by manual operation. In other words, the mobile terminal device TA executes processing of S39 when input operation of the submenu button 5111 is accepted by the TA nursing/care record processing portion 325 (Yes), and ends the present processing. In the processing of S39, each processing of the processing of S11 to the processing of S22 which have been described with reference to FIG. 11 is executed.

In the processing of S40, similarly to the above processing of S21, the mobile terminal device TA executes processing of a recording form to transmit contents of nursing/care input using the recording form displayed on the TA display unit 36 to the management server device SV as a nursing/care record by the TA nursing/care record processing portion 325, and thereafter, displays, on the TA display unit 36, the screen (the standby screen 51, the monitoring information screen 52, the nurse call reception screen 53, or the like) which has been displayed when accepting a recording form request in the processing of S31 to end the present processing.

Next, the nursing/care recording operation of the management server device SV in the second mode will be described. In FIG. 13, by means of the SV control portion 221 of the SV control processing unit 22, the management server device SV determines whether a communication signal is received by the SV communication IF unit 21 or not (S51). As a result of the determination, in a case where no communication signal is received (No), the management server device SV returns the processing to S51, and as a result of the determination, in a case where a communication signal is received (Yes), the management server device SV executes subsequent processing of S52. In other words, the management server device SV waits for reception of a communication signal.

In the processing of S52, the management server device SV discriminates the kind of the received communication signal by the SV control portion 221. As a result of the discrimination, in a case where the received communication signal is a first form request communication signal (first form request), the management server device SV, after execution of processing of S53, executes processing of S56, and in a case where the received communication signal is a second form request communication signal (second form request), after execution of the processing of S54, executes the processing of S56, and in a case where the received communication signal is neither the first nor the second form request communication signal (other), executes the processing of S56 after execution of the processing of S55.

In the processing of S53, the management server device SV executes, by the form distribution processing portion 223 of the SV control processing unit 22, first form return processing of returning a recording form on the basis of a status parameter in the terminal device SP, TA as a transmission source of the first form request communication signal received in the processing of S51 to the terminal device SP, TA as a transmission source of the first form request communication signal. More specifically, the form distribution processing portion 223 selects a recording form from among the plurality of recording forms stored in the form information storage portion 234 on the basis of the status parameter contained in the first form request communication signal received in the processing of S51, and transmits a first form return communication signal containing the selected recording form to the transmission source. More specifically, the form distribution processing portion 223 selects (searches), from the form information table FT stored in the form information storage portion 234, a record with the status parameter field 2341 in which the status parameter contained in the form request communication signal received in the processing of S51 is registered, extracts a recording form registered in the first candidate form sub-field 23421 in the recording form field 2342 of the selected record, and transmits a first form return communication signal containing the extracted recording form field to the transmission source. In more detail, in the present embodiment, a status parameter is composed of position information, monitor information, and reception time, of which the monitor information and the reception time are information that can be acquired by the management server device SV. Therefore, the form distribution processing portion 223 first acquires present year, month, day and time as reception time of the first form request communication signal received in the processing of S51, and extracts monitor information corresponding to the terminal ID contained in the first form request communication signal received in the processing of S51 from the SV storage unit 23. Next, the form distribution processing portion 223 selects (searches), from the form information table FT stored in the form information storage portion 234, a record in which position information contained in the first form request communication signal received in the processing of S51, the extracted monitor information, and the acquired reception time are registered in the place sub-field 23411, the monitor sub-field 23412, and the time sub-field 23413, respectively, and extracts a file name of a recording form registered in the first candidate form sub-field 23421 in the recording form field 2342 of the selected record.

Here, since information contained in the first form request communication signal as position information are an ID of the access point AP and a reception intensity thereof in the above example, in this case, a correspondence relationship (access point disposition position information) between a disposition position of the access point AP and the ID are stored in advance in the SV storage unit 23 in the management server device SV. With respect to each ID and a reception intensity thereof of the plurality of access points AP contained in the first form request communication signal received in the processing of S51, the form distribution processing portion 223 obtains a disposition position of the access point AP corresponding to the ID of the access point AP, and obtains a distance according to the reception intensity from the disposition position of the access point AP. And, the form distribution processing portion 223 obtains a position of the mobile terminal device TA as a transmission source of the first form request communication signal received in the processing of S51 as position information for use in the selection (search) on the basis of disposition positions of the plurality of access points AP and distances from the disposition positions according to the principle of so-called trigonometrical survey.

And, the form distribution processing portion 223 transmits a first form return communication signal containing an electronic file corresponding to a file name of the extracted recording form to the transmission source. Further, for recording reception of the first form return communication signal, and for use in processing of S54 to be described later, the form distribution processing portion 223 stores, in the SV storage unit 23, the terminal ID contained in the first form request communication signal received in the processing of S51, and the above-described position information, monitor information, and reception time obtained so as to be correlated with the terminal ID for a predetermined time period. The predetermined time is appropriate time required from the reception of a first form return communication signal until transmission of a second form request communication signal in the mobile terminal device TA, and is set to be, for example, 30 seconds, 1 minute, 3 minutes, etc.

In a case, for example, where position information, monitor information, and reception time are living room, care worker NB, and 9:30, respectively, the second row record is selected from the form information table FT shown in FIG. 6 to extract "breakfast", and an electronic file in a meal recording form corresponding to the "breakfast" is contained in a first form return communication signal to be transmitted to the transmission source. As a result, in the terminal device SP, TA, the above meal care recording form screen 64a shown in FIG. 17 is displayed on the TA display unit 36 in the above processing of S34, and use of the meal care recording form screen 64a in the above processing of S40 leads to input of contents of provided meal care as a nursing/care record.

In a case, for example, where position information, monitor information, and reception time are sitting room, care worker NA, and 7:00, respectively, the seventh row record is selected from the form information table FT shown in FIG. 6 to extract "morning care", and an electronic file in a morning care recording form corresponding to the "morning care" is contained in a first form return communication signal to be transmitted to the transmission source. As a result, in the terminal device SP, TA, a morning care recording form screen 64b shown, for example, in FIG. 18 is displayed on the TA display unit 36 in the above processing of S34, and use of the morning care recording form screen 64b in the above processing of S40 leads to input of contents of provided "morning care" as a nursing/care record.

The morning care recording form screen 64b is a screen on which a morning care recording form is displayed, the screen being for inputting the contents of conducted morning care as a nursing/care record. The morning care recording form screen 64b is provided with, as shown, for example, in FIG. 18, the menu bar region 511, the target person name display region 631, a nursing/care record input region 641b in which input items to be input as nursing/care contents are displayed to input nursing/care contents according to the input item as a nursing/care record, the sharing check box (□) 642, and the "transmit" button 643.

In the morning care recording form in the present embodiment, the input items are, for example, changing clothes, cosmetic formation, hairdressing, oral care, face-washing, get-up, bed-bathing, and other items, memos, and care provision time, and for inputting these items, the nursing/care record input region 641b has an item region 641b-1 including a changing clothes input check box (□) for inputting provision/non-provision of changing clothes, a cosmetic formation input check box (□) for inputting provision/non-provision of cosmetic formation, a hairdressing input check box (□) for inputting provision/non-provision of hairdressing, an oral care input check box (□) for inputting provision/non-provision of oral care, a face-washing input check box (□) for inputting provision/non-provision of face-washing, a get-up input check box (□) for inputting provision/non-provision of get-up, a bed-bathing input check box (□) for inputting provision/non-provision of bed-bathing, and other input check box (□) for inputting provision/non-provision of others, a free remarks input region 641b-2 for inputting memos, and a care provision time input region 641b-3 for inputting the time when nursing/care was provided. Input operation (e.g. tapping) of each region 641b-2, 641b-3 in the nursing/care record input region 641b of the morning care recording form screen 64b shown in FIG. 18 results in opening a sub-window for data input so as to be overlapped with the morning care recording form screen 64b, the sub-window being according to attributes (e.g. text data, time data, etc.) of data to be input to the relevant region 641b-2, 641b-3 similarly to the meal care recording form screen 64a.

As shown in FIG. 18, a default value of "present" is displayed in the care provision time input region 641b-3 similarly to the meal care recording form screen 64a (FIG. 17). In a case where the care provision time input region 641b-3 is not input by a monitor (user), the TA control portion 321 applies the default value of "present" as care provision time.

In a case, for example, where position information, monitor information, and reception time are toilet, care worker NA, and 11:15, respectively, the first row record is selected from the form information table FT shown in FIG. 6 to extract "excretion", and an electronic file in an excretion care recording form corresponding to the "excretion" is contained in a first form return communication signal to be transmitted to the transmission source. As a result, in the terminal device SP, TA, an excretion care recording form screen 64c shown, for example, in FIG. 19 is displayed on the TA display unit 36 in the above processing of S34, and use of the excretion care recording form screen 64c in the above processing of S40 leads to input of contents of provided excretion care as a nursing/care record.

The excretion care recording form screen 64c is a screen on which an excretion recording form is displayed, the screen being for inputting the contents of conducted excretion care as a nursing/care record. The excretion care recording form screen 64c is provided with, as shown, for example, in FIG. 19, the menu bar region 511, the target person name display region 631, a nursing/care record input region 641c in which input items to be input as nursing/care contents are displayed to input nursing/care contents according to the input item as a nursing/care record, the sharing check box (□) 642, and the "transmit" button 643.

In the excretion care recording form in the present embodiment, the input items are, for example, urination, defecation amount, defecation quality, memos, and care provision time, and for inputting these items, the nursing/care record input region 641c has an urination input region 641c-1 for inputting provision/non-provision of urination, a defecation amount input region 641c-2 for inputting a defecation amount, a defecation quality input region 641c-3 for inputting defecation quality, a free remarks input region 641c-4 for inputting memos, and a care provision time input region 641c-5 for inputting the time when nursing/care was provided. Input operation (e.g. tapping) of each of the regions 641c-1 to 641c-5 in the nursing/care record input region 641c of the excretion care recording form screen 64c shown in FIG. 19 results in opening a sub-window for data input so as to be overlapped with the excretion care recording form screen 64c, the sub-window being according to attributes (e.g. text data, time data, etc.) of data to be input to the relevant one of the regions 641c-1 to 641c-5 similarly to the meal care recording form screen 64a. For example, when input operation of the urination input region 641c-1 is conducted, an urination presence/absence selection screen 74 shown, for example, in FIG. 26, is displayed on the TA display unit 36 so as to be overlapped with the relevant excretion care recording form screen 64c. The urination presence/absence selection screen 74 is a screen for selecting and inputting presence/absence of urination to the relevant mobile terminal device TA. The urination presence/absence selection screen 74 has, as shown, for example, in FIG. 26, a urination presence/absence selection region 741 for displaying presence/absence of urination, and a "return" button 742 similar to the "return" button 712. Each region of the urination presence/absence selection region 741 in which presence or absence of urination is displayed is provided with a radio button (o) for designating and inputting either urination or no urination. In the example shown in FIG. 26, a radio button for no urination is checked to be designated and then input to the mobile terminal device TA.

As shown in FIG. 19, a default value of "present" is displayed in the care provision time input region 641c-5 similarly to the meal care recording form screen 64a (FIG. 17). In a case where the care provision time input region 641c-5 is not input by a monitor (user), the TA control portion 321 applies the default value of "present" as care provision time. Default values of care provision time shown in FIG. 17, FIG. 18, and FIG. 19 may be determined in advance for each type of care and stored in the TA storage unit 33 (one example of a storage unit).

Returning to FIG. 13, in the processing of S54, by means of the form distribution processing portion 223 of the SV control processing unit 22, the management server device SV conducts second form return processing of selecting a second recording form (second candidate recording form) different from the recording form (first candidate recording form) and returns the selected second recording form to the terminal device SP, TA as the transmission source, the recording form being selected for the first form return communication signal on the basis of the status parameter contained in the first form request communication signal derived from transmission of the first form return communication signal transmitted in the processing of S53. More specifically, the form distribution processing portion 223 selects a second recording form different from the recording form selected for the first form return communication signal in the processing of S53 from among the plurality of recording forms stored in the form information storage portion 234 on the basis of the status parameter contained in the first form request communication signal derived from transmission of the first form return communication signal transmitted in the processing of S53, and transmits a second form return communication signal containing the selected second recording form to the transmission source. More specifically, the form distribution processing portion 223 selects (searches), from the form information table FT stored in the form information storage portion 234, a record with the status parameter field 2341 in which the status parameter contained in the first form request communication signal derived from the transmission of the first form return communication signal transmitted in the processing of S53 is to be registered, extracts a recording form registered in the second candidate form sub-field 23422 in the recording form field 2342 of the selected record, and transmits a second form return communication signal containing the extracted recording form to the transmission source. In more detail, in the present embodiment, first, in a case where the terminal ID contained in the second form request communication signal received in the processing of S51 is stored in the SV storage unit 23 as the terminal ID having received the first form request communication signal, the form distribution processing portion 223 extracts position information, monitor information, and reception time correlated with the terminal ID from the SV storage unit 23. Next, the form distribution processing portion 223 selects (searches) a record with the place sub-field 23411, the monitor sub-field 23412, and the time sub-field 23413 in which these extracted position information, monitor information, and reception time are registered, respectively, from the form information table FT stored in the form information storage portion 234, and extracts a file name in a recording form registered in the second candidate form sub-field 23422 in the recording form field 2342 of the selected record. And, the form distribution processing portion 223 transmits a second form return communication signal containing an electronic file corresponding to the extracted file name in the recording form to the transmission source.

In the above example, in a case where position information, monitor information, and reception time extracted from the SV storage unit 23 are, for example, living room, care worker NB, and 9:30, respectively, the second row record is selected from the form information table FT shown in FIG. 6 to extract "taking-medicine", and an electronic file in a taking-medicine recording form corresponding to the "taking-medicine" is contained in a second form return communication signal to be transmitted to the transmission source. As a result, in the terminal device SP, TA, a taking-medicine care recording form screen not shown is displayed on the TA display unit 36 in the above processing of S37, and use of the taking-medicine care recording form screen not shown in the above processing of S40 leads to input of contents of provided taking-medicine care as a nursing/care record.

In the above example, in a case where position information, monitor information, and reception time extracted from the SV storage unit 23 are, for example, sitting room, care worker NA, and 7:00, respectively, the seventh row record is selected from the form information table FT shown in FIG. 6 to extract "excretion", and an electronic file in an excretion recording form corresponding to the "excretion" is contained in a second form return communication signal to be transmitted to the transmission source. As a result, in the terminal device SP, TA, the excretion care recording form screen 64c shown, for example, in FIG. 19 is displayed on the TA display unit 36 in the above processing of S37, and use of the excretion care recording form screen 64c in the above processing of S40 leads to input of contents of provided excretion care as a nursing/care record.

In the above example, in a case where position information, monitor information, and reception time extracted from the SV storage unit 23 are, for example, toilet, care worker NA, and 11:15, respectively, the first row record is selected from the form information table FT shown in FIG. 6 to extract "tooth-brushing", and an electronic file in a tooth-brushing care recording form corresponding to the "tooth-brushing" is contained in a second form return communication signal to be transmitted to the transmission source. As a result, in the terminal device SP, TA, a tooth-brushing care recording form screen not shown is displayed on the TA display unit 36 in the above processing of S37, and use of the tooth-brushing care recording form screen not shown in the above processing of S40 leads to input of contents of provided tooth-brushing care as a nursing/care record.

In a case where the terminal ID contained in the second form request communication signal received in the processing of S51 is not stored in the SV storage unit 23 as the terminal ID having received the first form request communication signal, due to a lapse of the predetermined time, the form distribution processing portion 223 executes predetermined error processing. The predetermined error processing is, for example, processing of transmitting, to the mobile terminal device TA as a transmission source, a communication signal (form selection screen display instruction communication signal) containing an instruction to cause the relevant mobile terminal device TA to display the form selection screen 63, or other processing.

In the processing of S55, the management server device SV executes appropriate processing according to the communication signal received in the processing of S51 by the SV control processing unit 22. For example, upon receiving the nursing/care record communication signal, the management server device SV stores a target person name (monitored person name) and contents of a nursing/care record in the nursing/care record information storage portion 235 so as to be correlated with each other as described above by the SV nursing/care record processing portion 224 of the SV control processing unit 22, the target person name and the contents of a nursing/care record being contained in the received nursing/care record communication signal.

And, in the processing of S56 executed after each of the processing of S53 to the processing of S55, the management server device SV determines whether operation thereof ends (stops) or not by the SV control processing unit 22. As a result of the determination, in a case where the operation ends (stops) (Yes), the management server device SV ends the present processing, while in a case where the operation does not end (stop) (No), the management server device SV returns the processing to the processing of S51.

In the nursing/care recording operation in the second mode, each processing described above is executed, resulting in inputting a nursing/care record to the mobile terminal device TA using a recording form and in storing (recording) the same in the management server device SV.

As described in the foregoing, in the monitored person monitoring system MS, the management server device SV as one example of the monitored person monitoring device, and the monitored person monitoring method mounted on the same according to the present embodiment, upon reception of a first form request communication signal, a recording form (first candidate recording form) is selected from among the plurality of recording forms on the basis of a status parameter contained in the received first form request communication signal, and a first form return communication signal containing the selected recording form is transmitted to a transmission source. Accordingly, since the terminal device SP, TA as a transmission source displays a recording form on the basis of a status parameter, a recording form suitable for the predetermined status is displayed on the terminal device SP, TA as the transmission source. Since it is expected that a user (monitor) handling the terminal device SP, TA as the transmission source wants a recording form according to a predetermined status in some cases, it is highly probable that a recording form suitable for the predetermined status is a recording form wanted by the user. Therefore, the above-described monitored person monitoring system, management server device SV and method thereof more efficiently provide a user's desired form while saving labor for selecting a desired form.

Also, in the above, there might occur a case where a recording form (first candidate recording form) which has been contained, deriving from a first form request communication signal, in a first form return communication signal and transmitted to a transmission source is not a recording form wanted by a user. In such a case, in the above monitored person monitoring system, management server device SV, and method thereof, upon reception of a second form request communication signal, a second recording form (second candidate recording form) different from the selected recording form (first candidate recording form) is selected from among the plurality of recording forms on the basis of a status parameter contained in the received first form request communication signal, and a second form return communication signal containing the selected second recording form is transmitted to a transmission source. It is more highly probable that the second recording form is a recording form wanted by the user. Therefore, the above-described monitored person monitoring system, management server device SV and method thereof more efficiently provide a user's desired form while saving labor for selecting a desired form.

Also, in the above-described monitored person monitoring system, management server device SV and method thereof, since the status parameter is at least one of position information indicative of a position of the transmission source, monitor information indicative of a monitor who handles a device as the transmission source, and reception time of the form request communication signal, it is highly probable that a recording form on a basis of a status parameter is a recording form wanted by the user. Therefore, the above-described monitored person monitoring system, management server device SV and method thereof more efficiently provide a user's desired form while saving labor for selecting a desired form.

Additionally, according to the present embodiment, when a default value displayed in the care provision time input region 641a-4 is selected as an input target from a plurality of nursing/care items included in the nursing/care record input region 641a, the time selection screen 75 is displayed on the TA display unit 36 as an input screen, the time selection screen displaying a plurality of different elapsed times dating back from current time as options of nursing/care provision time. Therefore, selection from a plurality of elapsed times leads to input of care provision time, thereby realizing simplification of care provision time input work.

### (Others)

(1) In the above embodiment, as shown in FIG. 17 to FIG. 19, a default value of "present" is displayed in the care provision time input region of each of the recording form screens 64a, 64b, and 64c, and in a case where input to the care provision time input region is not conducted by a monitor (user), the default value of "present" is applied as care provision time.

In general, average time required for a monitor (user) to proceed to input work for recording care after provision of the care varies with the type of care. For example, in a case of dining, some amount of time is required for clearing table etc., and therefore, it is impossible to proceed to input work immediately after provision of care. However, regarding taking-medicine or hydration, etc., it is possible to proceed to input work immediately after provision of care. Accordingly, a default value ("present" in the example of FIG. 17 to FIG. 19) may be changed for each type of care.

Specifically, for example, the TA control portion 321 may display a default value of "15 minutes ago" in the care provision time input region 641a-4 on the meal care recording form screen 64a shown in FIG. 17, and in a case where input to the care provision time input region 641a-4 is not conducted by a monitor (user), the default value of "15 minutes ago" may be applied as care provision time.

Also, for example, the TA control portion 321 may display a default value of "20 minutes ago" in the care provision time input region 641b-3 of the nursing/care record input region 641b on the morning care recording form screen 64b shown in FIG. 18, and in a case where no input to the care provision time input region 641b-3 is conducted, the default value of "20 minutes ago" may be applied as care provision time.

Also, for example, the TA control portion 321 may display a default value of "ten minutes ago" in the care provision time input region 641c-5 of the nursing/care record input region 641c on the excretion care recording form screen 64c shown in FIG. 19, and in a case where no input to the care provision time input region 641c-5 is conducted, the default value of "ten minutes ago" may be applied as care provision time.

Also, for example, the TA control portion 321 may display a default value of "five minutes ago" in the care provision time input region on a hydration care recording form screen (not shown) and on taking-medicine care recording form screen (not shown), and in a case where no input to the care provision time input region is conducted, the default value of "five minutes ago" may be applied as care provision time.

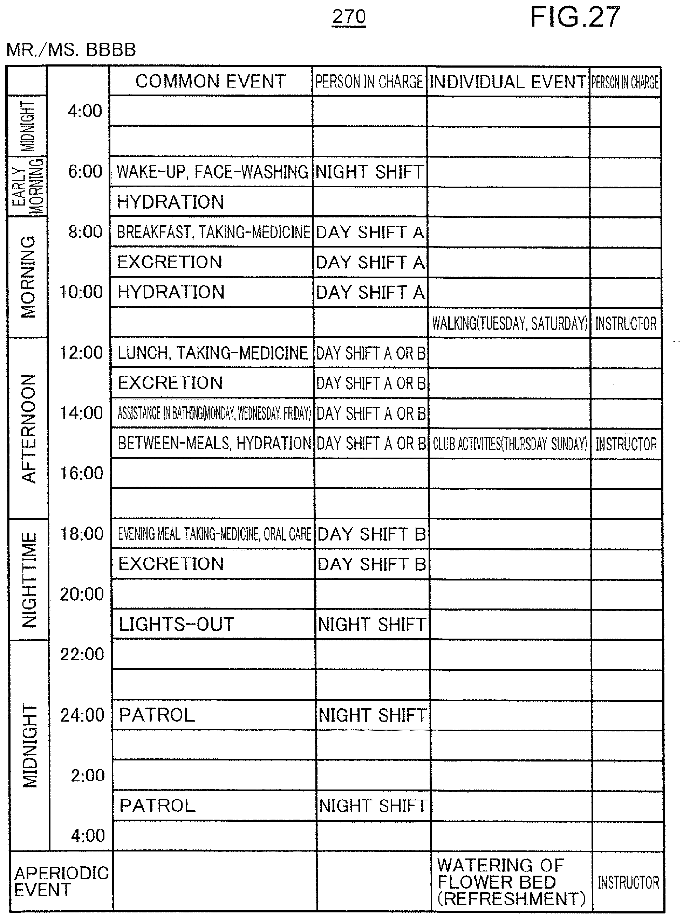

(2) In the above-described embodiment, although the default value is a fixed value such as "present" as shown in FIG. 17 for example, the default value is not limited thereto but may be a variable value. The SV storage unit 23 (one example of a nursing/care plan storage portion) of the management server device SV shown in FIG. 2 may store a care plan (one example of a nursing/care plan) shown, for example, in FIG. 27.

FIG. 27 is a view schematically showing one example of a care plan 270 stored in the SV storage unit 23 of the management server device SV.

The care plan 270 is a schedule in which scheduled care provision time for each monitored person Ob is recited. The care plan 270 has recitation of, for example, meal care such as breakfast, lunch, and evening meal, excretion care, hydration care, etc. The example of FIG. 27 shows the care plan 270 for Mr./Ms. BBBB in room No. 102 (FIG. 5).

The SV control portion 221 of the management server device SV may therefore determine a default value of care provision time on the basis of the scheduled care provision time recited in the care plan 270.

As described above, for example, when input operation of the region (form selection button) 632-1 in which "meal" is displayed is conducted in the processing of S18 in FIG. 11, the mobile terminal device TA transmits a third form request communication signal for requesting a recording form for meal care to the management server device SV via the SV communication IF unit 21 by means of the TA nursing/care record processing portion 325 (one example of a request control portion) (S19). The management server device SV transmits a third return form communication signal containing an electronic file in the meal care recording form to the mobile terminal device TA as a response.

At this time, the SV control portion 221 of the management server device SV reads the care plan 270 of the relevant monitored person Ob from the care plan 270 for each monitored person Ob stored in the SV storage unit 23. The SV control portion 221 extracts scheduled provision time for the relevant care from the care plan 270. In the example of FIG. 27, in a case where care is breakfast for example, the SV control portion 221 extracts "8" from the care plan 270. The SV control portion 221 calculates, on a minute basis, how many minutes ago (i.e. dating back elapsed time), "8" extracted from the care plan 270 falls on from current time (e.g. time when the third form request communication signal transmitted by the mobile terminal device TA in the processing of S19 shown in FIG. 11 is received by the SV communication IF unit 21 of the management server device SV).

The SV control portion 221 transmits the third return form communication signal with the calculated elapsed time, for example, "○○ minutes ago" contained therein as a default value, to the mobile terminal device TA. The TA control portion 321 of the mobile terminal device TA displays "○○ minutes ago" in the care provision time input region 641a-4 on the meal care recording form screen 64a as the default value transmitted from the management server device SV.

The management server device SV may transmit an approximate value obtained by approximating, for example, on the basis of ten minutes or on the basis of 15 minutes, the elapsed time calculated on a minute basis to the mobile terminal device TA as a default value. In this case, a default value is displayed on the basis of ten minutes or on the basis of 15 minutes, such as "20 minutes ago", "30 minutes ago", etc. in the care provision time input region 641a-4 on the meal care recording form screen 64a.

When a default value of the care provision time is thus changed on the basis of the scheduled provision time of the care plan 270, in a case where the care provision time is coincident with the scheduled provision time of the care plan 270, a possibility that a monitor (user) need not change a care provision time from the default value is increased. This increases a possibility that a load of input work on a monitor (user) is mitigated.
(3) In the above-described embodiment, although the status parameter is composed of position information, monitor information, and reception time, the status parameter may be any one of the position information, the monitor information, and the reception time, or any two of the position information, the monitor information, and the reception time. Further, the status parameter may include, in addition to these position information, monitor information, and reception time, or in place of the same, another parameter such as a previously displayed recording form, or the like.
(4) In the above-described embodiment, although the first form request communication signal contains position information as a status parameter, the signal may contain position information and monitor information, or may contain position information, monitor information, and transmission time. The transmission time is used as request time of the recording form in place of the reception time.
(5) In the above-described embodiment, although a recording form automatically displayed in the terminal device SP, TA includes two candidates including first and second candidates, the recording form may include three or more candidates.
(6) In the above-described embodiment, although taking medicine by a monitored person Ob is referred to as "taking-medicine", giving medicine to a monitored person Ob who is nursed is referred to as "medication" in general.

The present specification discloses techniques of various modes as described above, of which main techniques will be summarized in the following.

According to a first aspect, the present invention relates to a nursing/care item input device which is used to input results of nursing/care regarding a plurality of nursing/care items determined in advance for each type of nursing/care, the nursing/care to be provided for a monitored person as a monitoring target, the nursing/care including at least one of nursing and care, the nursing/care item input device including:
a display unit which displays a display screen including the plurality of nursing/care items;
an operation input unit which accepts operation input by a user; and
a display control portion which displays on the display unit, when one nursing/care item is selected as an input target from the plurality of nursing/care items by the user using the operation input unit in a state where the display screen is displayed on the display unit, an input screen for inputting a result of nursing/care regarding the selected one nursing/care item, wherein
one of the plurality of nursing/care items is nursing/care provision time indicative of a time when the nursing/care is provided, and
the display control portion displays on the display unit, when the nursing/care provision time is selected as an input target from the plurality of nursing/care items, a screen indicating a plurality of different elapsed times dating back from current time as options of the nursing/care provision time as the input screen.

According to the first aspect, in a state where a display screen including a plurality of nursing/care items is displayed on the display unit, when one nursing/care item is selected as an input target from the plurality of nursing/care items by a user, an input screen is displayed on the display unit, the input screen to be used for inputting a result of provision of nursing/care regarding the selected one nursing/care item. One of the plurality of nursing/care items is nursing/care provision time indicative of the time when nursing/care was provided. When the item nursing/care provision time is selected as an input target from the plurality of nursing/care items, a screen is displayed on the display unit as an input screen, the screen being indicative of a plurality of different elapsed times dating back from current time as options of nursing/care provision time.

According to the first aspect, therefore, nursing/care provision time can be input by selection from different elapsed times dating back from current time. Further, since elapsed time dating back from current time is represented, for example, as "○○ minutes ago", it is only necessary to sensuously memorize, for example, "○○ minutes ago" as the time when nursing/care was provided. It is therefore possible to easily select correct elapsed time without mistakes. In addition, it is not necessary to check current time at the time of input of nursing/care provision time. Even in a case of looking at a clock to check the time when nursing/care was provided, it is only necessary to seize elapsed time dating back from current time not on an hour basis but on a minute basis (e.g. by looking at a long hand in a case of an analog watch). As a result, according to the first aspect, simplification of nursing/care provision time input work can be realized.

In the above first aspect, for example,
the display control portion may display on the display unit, when the nursing/care provision time is selected as an input target, a screen indicating options enabling a numerical value of time to be input in addition to the options of the nursing/care provision time as the input screen.

In the present aspect, when the nursing/care provision time is selected as an input item, a screen is displayed as an input screen on the display unit, the screen indicating options enabling a numerical value of time to be input in addition to options of the nursing/care provision time. Accordingly, in a case where an appropriate elapsed time is not included in a plurality of different elapsed times dating back from current time, selecting an option which enables input of a numerical value of time allows input of nursing/care provision time.

In the above first aspect, for example,
a storage unit may be further provided which stores a default value of the nursing/care provision time determined in advance for each type of the nursing/care.

In the above first aspect, for example,
a storage unit may be further provided which stores a default value that is determined in advance for each type of the nursing/care and that is applied as the nursing/care provision time in a case where the nursing/care provision time is not selected using the operation input unit.

The display unit may display a screen including the default value as the display screen.

The display control portion may display the input screen on the display unit when the default value included in the display screen is selected by the user using the operation input unit.

In the present aspect, a default value is stored in the storage unit, the default value being determined in advance for each type of nursing/care and being applied as nursing/care provision time in a case where nursing/care provision time is not selected by the operation input unit. A screen including a default value is displayed on the display unit as a display screen. When the default value included in the display screen is selected by a user by using the operation input unit, an input screen is displayed on the display unit. Accordingly, when the input screen is displayed on the display unit, nursing/care provision time can be selected from among a plurality of different elapsed times dating back from current time.

By contrast, in a case where the default value included in the display screen is not selected by a user using the operation input unit, no input screen is displayed on the display unit, and the default value is applied as nursing/care provision time. Accordingly, in a case where a default value is applicable as nursing/care provision time, a user needs no selection of a default value using the operation input unit. As a result, nursing/care provision time input work can be further simplified.

According to a second aspect, the present invention relates to a nursing/care item input method for use in a nursing/care item input device which is used to input results of nursing/care regarding a plurality of nursing/care items determined in advance for each type of nursing/care, the nursing/care to be provided for a monitored person as a monitoring target, the nursing/care including at least one of nursing and care, the nursing/care item input method including:
a displaying step of displaying a display screen including the plurality of nursing/care items on a display unit;
an accepting step of accepting user's selection of one nursing/care item as an input target from the plurality of nursing/care items using an operation input unit, in a state where the display screen is displayed on the display unit; and
a display controlling step of displaying, upon accepting the user's selection in the accepting step, an input screen for inputting a result of nursing/care regarding the selected one nursing/care item on the display unit, wherein
one of the plurality of nursing/care items is nursing/care provision time indicative of a time when the nursing/care is provided, and
the display controlling step includes displaying on the display unit, when the nursing/care provision time is selected as an input target from the plurality of nursing/care items, a screen indicating a plurality of different elapsed times dating back from current time as options of the nursing/care provision time as the input screen.

In the second aspect, user's selection of one nursing/care item is accepted as an input target from a plurality of nursing/care items in a state where a display screen including the plurality of nursing/care items is displayed on the display unit. Upon acceptance of the user' selection, an input screen for inputting a result of nursing/care regarding the selected one nursing/care item is displayed on the display unit. One of the plurality of nursing/care items is nursing/care provision time indicative of the time when the nursing/care was provided. When nursing/care provision time is selected as an input target from the plurality of nursing/care items, a screen is displayed on the display unit, the screen indicating a plurality of different elapsed times dating back from current time as options of the nursing/care provision time as an input screen.

According to the second aspect, therefore, nursing/care provision time can be input by selection from different elapsed times dating back from current time. Further, since elapsed time dating back from current time is represented, for example, as "○○ minutes ago", it is only necessary to sensuously memorize, for example, "○○ minutes ago" as the time when nursing/care was provided. It is therefore possible to easily select correct elapsed time without mistakes. In addition, it is not necessary to check current time at the time of input of nursing/care provision time. Even in a case of looking at a clock to check the time when nursing/care was provided, it is only necessary to seize elapsed time dating back from current time not on an hour basis but on a minute basis (e.g. by looking at a long hand in a case of an analog watch). As a result, according to the second aspect, simplification of nursing/care provision time input work can be realized.

According to a third aspect, the present invention relates to a nursing/care item management system including:
the nursing/care item input device according to the above aspect; and
a management server device which stores and manages, for each type of the nursing/care, results of nursing/care regarding the plurality of nursing/care items input in the nursing/care item input device, wherein
the nursing/care item input device further includes:
   an input side communication unit which communicates with the management server device; and
   a request control portion which requests a recording form for forming a display screen including the plurality of nursing/care items according to types of the nursing/care from the management server device via the input side communication unit,
the management server device includes:
   a server side communication unit which communicates with the nursing/care item input device;
   a nursing/care plan storage portion which stores a nursing/care plan indicating scheduled time for provision of each type of the nursing/care for the monitored person; and
   a response control portion which extracts, upon reception of the request for the recording form via the server side communication unit, scheduled time corresponding to the type of the nursing/care from the nursing/care plan, calculates elapsed time dating back from reception time when the request for the recording form is received to the extracted scheduled time, and transmits the recording form including the calculated elapsed time as a default value to the nursing/care item input device,
the default value is applied as the nursing/care provision time in a case where the nursing/care provision time is not selected using the operation input unit,
the display unit makes up the display screen using the recording form, and
the display screen includes the elapsed time calculated by the response control portion as the default value.

In the third aspect, upon reception of a request for a default value corresponding to the type of nursing/care transmitted from the nursing/care item input device, scheduled time corresponding to the type of nursing/care is extracted from a nursing/care plan, elapsed time dating back from reception time when the request for a default value is received to the extracted scheduled time is calculated, and the calculated elapsed time is transmitted to the nursing/care item input device. A display screen including the calculated elapsed time as a default value is displayed on the display unit.

Accordingly, in a case where nursing/care for a monitored person was conducted at scheduled time indicated in a nursing/care plan, the calculated elapsed time can be taken as nursing/care provision time. Therefore, it is not necessary to change nursing/care provision time from a default value. As a result, in a case where nursing/care for a monitored person was conducted at scheduled time indicated in the nursing/care plan, nursing/care provision time input work can be saved.

Although the display unit may display the elapsed time calculated by the response control portion, as it is, as a screen including the default value, a screen may be displayed which includes, as the default value, time obtained by approximating the elapsed time calculated by the response control portion, for example, on the basis of ten minutes or on the basis of 15 minutes.

According to a fourth aspect, the present invention relates to a nursing/care item management system including:
the nursing/care item input device according to the above aspect; and
a management server device which includes a server side storage portion which stores results of nursing/care regarding the plurality of nursing/care items input in the nursing/care item input device for each type of the nursing/care, wherein
the nursing/care item input device further includes:
   an input side communication unit which communicates with the management server device; and
   a transmission control portion which transmits, when one elapsed time is selected from the plurality of elapsed times by the user using the operation input unit in a state where the input screen is displayed on the display unit, the selected one elapsed time to the management server device, and
the management server device includes:
   a server side communication unit which communicates with the nursing/care item input device; and
   a management control portion which causes, upon reception of the selected one elapsed time that is transmitted from the nursing/care item input device via the server side communication unit, the server side storage portion to store time dating back by the selected one elapsed time from the reception time as the nursing/care provision time.

In the fourth aspect, upon reception of selected one elapsed time that is transmitted from the nursing/care item input device, time dating back by the selected one elapsed time from the reception time is stored in the server side storage portion as the nursing/care provision time. Accordingly, according to the fourth aspect, nursing/care provision time can be precisely managed.

Although for expressing the present invention, the present invention has been appropriately and sufficiently described through the embodiments with reference to the drawings in the foregoing, it should be recognized that those skilled in the art could change and/or improve the above embodiments with ease. Accordingly, as long as a changed mode or an improved mode made by those skilled in the art are within the scope of right recited in claims, the changed mode or improved mode are construed to be encompassed in the scope of claims.

The entire disclosure of Japanese Patent Application No. 2016-127261, filed on June 28, 2016, including the specification, the claims, drawings, and abstract, is incorporated herein by reference in its entirety.

While the embodiments of the present invention have been described in detail, the embodiments of the present invention are for illustrative purpose only and not to be construed as limiting in any manner, and should be construed only by attached claim words.

## Claims

1. A nursing/care item input device which is used to input results of nursing/care regarding a plurality of nursing/care items determined in advance for each type of nursing/care, the nursing/care to be provided for a monitored person as a monitoring target, the nursing/care including at least one of nursing and care, the nursing/care item input device comprising:
a display unit which displays a display screen including the plurality of nursing/care items;
an operation input unit which accepts operation input by a user; and
a display control portion which displays on the display unit, when one nursing/care item is selected as an input target from the plurality of nursing/care items by the user using the operation input unit in a state where the display screen is displayed on the display unit, an input screen for inputting a result of nursing/care regarding the selected one nursing/care item, wherein
one of the plurality of nursing/care items is nursing/care provision time indicative of a time when the nursing/care is provided, and
the display control portion displays on the display unit, when the nursing/care provision time is selected as an input target from the plurality of nursing/care items, a screen indicating a plurality of different elapsed times dating back from current time as options of the nursing/care provision time as the input screen.

2. The nursing/care item input device according to claim 1, wherein the display control portion displays on the display unit, when the nursing/care provision time is selected as an input target, a screen indicating options enabling a numerical value of time to be input in addition to the options of the nursing/care provision time as the input screen.

3. The nursing/care item input device according to claim 1 or 2, further comprising a storage unit which stores a default value of the nursing/care provision time determined in advance for each type of the nursing/care.

4. The nursing/care item input device according to claim 1 or 2, further comprising a storage unit which stores a default value that is determined in advance for each type of the nursing/care and that is applied as the nursing/care provision time in a case where the nursing/care provision time is not selected using the operation input unit, wherein
the display unit displays a screen including the default value as the display screen, and
the display control portion displays the input screen on the display unit when the default value included in the display screen is selected by the user using the operation input unit.

5. A nursing/care item input method for use in a nursing/care item input device which is used to input results of nursing/care regarding a plurality of nursing/care items determined in advance for each type of nursing/care, the nursing/care to be provided for a monitored person as a monitoring target, the nursing/care including at least one of nursing and care, the nursing/care item input method comprising:
a displaying step of displaying a display screen including the plurality of nursing/care items on a display unit;
an accepting step of accepting user's selection of one nursing/care item as an input target from the plurality of nursing/care items using an operation input unit, in a state where the display screen is displayed on the display unit; and
a display controlling step of displaying, upon accepting the user's selection in the accepting step, an input screen for inputting a result of nursing/care regarding the selected one nursing/care item on the display unit, wherein
one of the plurality of nursing/care items is nursing/care provision time indicative of a time when the nursing/care is provided, and
the display controlling step includes displaying on the display unit, when the nursing/care provision time is selected as an input target from the plurality of nursing/care items, a screen indicating a plurality of different elapsed times dating back from current time as options of the nursing/care provision time as the input screen.

6. A nursing/care item management system comprising:
the nursing/care item input device according to claim 1 or 2; and
a management server device which stores and manages, for each type of the nursing/care, results of nursing/care regarding the plurality of nursing/care items input in the nursing/care item input device, wherein
the nursing/care item input device further includes:
an input side communication unit which communicates with the management server device; and
a request control portion which requests a recording form for forming a display screen including the plurality of nursing/care items according to types of the nursing/care from the management server device via the input side communication unit,
the management server device includes:
a server side communication unit which communicates with the nursing/care item input device;
a nursing/care plan storage portion which stores a nursing/care plan indicating scheduled time for provision of each type of the nursing/care for the monitored person; and
a response control portion which extracts, upon reception of the request for the recording form via the server side communication unit, scheduled time corresponding to the type of the nursing/care from the nursing/care plan, calculates elapsed time dating back from reception time when the request for the recording form is received to the extracted scheduled time, and transmits the recording form including the calculated elapsed time as a default value to the nursing/care item input device,
the default value is applied as the nursing/care provision time in a case where the nursing/care provision time is not selected using the operation input unit,
the display unit makes up the display screen using the recording form, and
the display screen includes the elapsed time calculated by the response control portion as the default value.

7. A nursing/care item management system comprising:
the nursing/care item input device according to any one of claims 1 to 4; and
a management server device which includes a server side storage portion which stores results of nursing/care regarding the plurality of nursing/care items input in the nursing/care item input device for each type of the nursing/care, wherein
the nursing/care item input device further includes:
an input side communication unit which communicates with the management server device; and
a transmission control portion which transmits, when one elapsed time is selected from the plurality of elapsed times by the user using the operation input unit in a state where the input screen is displayed on the display unit, the selected one elapsed time to the management server device, and
the management server device includes:
a server side communication unit which communicates with the nursing/care item input device; and
a management control portion which causes, upon reception of the selected one elapsed time that is transmitted from the nursing/care item input device via the server side communication unit, the server side storage portion to store time dating back by the selected one elapsed time from the reception time as the nursing/care provision time.
